Europäisches Patentamt

(19) European Patent Office

Office européen des brevets

(11) Numéro de publication : **0 412 912 B1**

(12) **FASCICULE DE BREVET EUROPEEN**

(45) Date de publication du fascicule du brevet :
**16.03.94 Bulletin 94/11**

(21) Numéro de dépôt : **90402282.9**

(22) Date de dépôt : **09.08.90**

(51) Int. Cl.$^5$ : **C12N 15/82, A01H 4/00,
C12N 5/00, A01H 5/00,
C12N 15/40, C12N 5/04**

(54) **Plantes transgéniques appartenant à l'espèce Cucumis melo.**

Le dossier contient des informations techniques présentées postérieurement au dépôt de la demande et ne figurant pas dans le présent fascicule.

(30) Priorité : **11.08.89 FR 8910848**

(43) Date de publication de la demande :
**13.02.91 Bulletin 91/07**

(45) Mention de la délivrance du brevet :
**16.03.94 Bulletin 94/11**

(84) Etats contractants désignés :
**BE DE ES FR GB GR IT LU NL**

(56) Documents cités :
EP-A- 0 257 993
WO-A-89/05858
BIOTECHNOLOGY, vol. 6, mai 1988, pages 549-557; M. CUOZZO et al.: "Viralprotection in transgenic tobacco plants expressing the cucumber mosaic viruscoat protein or its anti-sense RNA"
IDEM
BIOLOGICAL ABSTRACTS, vol. 87, 1989, abrégé no. 12323, Philadelphia, PA, US; J.OWEN et al.: "Characterization of cucumber mosaic virus: I. Molecularheterogeneity mapping of RNA 3 in eight CMV strains", & VIROLOGY 166(2): 495-502. 1988
BIOLOGICAL ABSTRACTS/RMM, abrégé BR37:95629, Philadelphia, PA, US; G. FANG etal.: "Agrobacterium-tumefaciens mediated-transformation and regeneration of meloncu-cumis-melo L"
BIOLOGICAL ABSTRACTS, vol. 79, no. 11, 1985, résumé no. 95343, Philadelphia,PA, US; C.K. KREUTMEIER et al.: "The effect of magnesium sulfate and calciumchloride on regeneration of shoot tip cultures of Prunus cerasus in vitro", & GARTENBAUWISSENSCHAFT 49(5/6): 204-212. 1984[recd. 1985]

(56) Documents cités :
NUCLEIC ACIDS RESEARCH, vol. 18, no. 5, 11 mars 1990, page 1332; M.J.T. NOEL etal.: "Nucleotide sequence of the coat protein gene and flanking regions ofcucumber virus (CMV) strain 117F"
Moreno et al. Plant Cell Tissue Organ Culture Vol. 5, 1985 pp. 139-146
Ezura et al., Plant Science, Vol. 85, 1992 pp.209-213
Ezura et al., Japan J. Breed. vol 42, 1992 pp. 137-144 (English abstract)

(73) Titulaire : **BIOCEM**
**24, avenue des Landais, Campus Universitaire des Cezeaux**
**F-63170 Aubière (FR)**

(72) Inventeur : **De Both, Michiel**
**11, rue St-Exupéry**
**F-63110 Beaumont (FR)**
Inventeur : **Ben Tahar, Sophia**
**25, rue Vernemouze**
**F-63000 Clermont-Ferrand (FR)**
Inventeur : **Noel, Marianne**
**30, rue du Cheval Blanc**
**F-63000 Clermont-Ferrand (FR)**
Inventeur : **Perret, Joel**
**26, Chemin de Giroux, Opme**
**F-63540 Romagnat (FR)**

(74) Mandataire : **Gutmann, Ernest et al**
**Ernest Gutmann - Yves Plasseraud S.A. 3, rue Chauveau-Lagarde**
**F-75008 Paris (FR)**

Jouve, 18, rue Saint-Denis, 75001 PARIS

## Description

Cette invention décrit un procédé de transformation génétique de plantes appartenant au genre Cucumis, et en particulier du melon (Cucumis melo). Ce procédé implique la transformation d'explants par Agrobacterium tumefaciens et la régénération, in vitro, des plantes transformées.

Cette technique de transformation et régénération peut être utilisée pour introduire par exemple un gène de résistance au virus de la mosaïque du concombre dans des plantes appartenant à l'espèce cucumis melo.

Le transfert des caractères génétiques d'une espèce végétale à une autre est limité, dans de nombreux cas, par des barrières d'incompatibilité.

Ces problèmes se rencontrent particulièrement pour la famille des Cucurbitaceae, pour les genres Cucumis (le melon et le concombre ) et Cucurbita (la courge). Ainsi le transfert de caractères agronomiquement intéressants tels que les résistances aux maladies virales ou aux insectes, présents chez des espèces sauvages, ne peuvent être transférés aux espèces cultivées.

Parmi les espèces cultivées du genre Cucumis, les seuls croisements sexuels possibles se font entre C. sativus (le concombre) et les espèces voisines C. hardwickii et C. Sikkimensis (Deakin et al, 1971; Van Raamsdonk, 1989). Les croisements de C. sativus avec d'autres espèces sauvages ne donneront, dans la plupart des cas, que des fruits stériles ; cependant, le melon (Cucumis melo) se présente comme une espèce récalcitrante qui ne peut être croisée avec aucune autre espèce (Kho et al, 1980; Van Raamsdonk, 1989).

Les applications des nouvelles techniques du génie génétique présentent une alternative prometteuse pour l'introduction de caractères nouveaux en vue de l'amélioration des espèces végétales. Parmi ces techniques on trouvera la transformation génétique par l'introduction d'un gène ou de gènes étrangers, l'hybridation somatique par fusion de protoplastes et l'induction de variations somaclonales ou de mutations pour induire des modifications génétiques.

Le transfert de gènes étrangers dans des espèces végétales se fait de façon assez courante en utilisant des souches d'Agrobacterium tumefaciens contenant un plasmide Ti désarmé (Fraley et al, 1986) (Klee et al, 1987) (Horsch et al, 1985). Actuellement une grande variété de plantes transgéniques a été obtenue avec Agrobacterium tumefaciens. Cette bactérie permet de transférer un gène étranger à des cellules végétales qui pourront régénérer des plantes transformées. Des gènes codant pour des caractères agronomiques intéressants ont été introduits dans des espèces végétales. Ainsi des plantes résistantes aux herbicides (Degreef et al, 1989) et aux insectes (Hilder et al, 1987 ; Vaeck et al, 1987) ont pu être obtenues.

Le transfert de gènes peut se faire soit à partir du plasmide Ti désarmé, après recombinaison homologue, en utilisant des vecteurs intermédiaires (Fraley et al, 1985) soit à partir d'un vecteur binaire à l'aide d'un plasmide Ti désarmé (Bevan, 1984 ; Fraley et al, 1986).

Ce transfert de gènes peut aussi s'opérer par l'utilisation d'Agrobacterium rhizogenes qui induira à partir du tissu transformé des racines au lieu de plantes transgéniques. A partir de ces racines transformées, des plantes ayant un phénotype anormal peuvent être régénérées (Chilton et al, 1982, David et al, 1984).

Un caractère particulièrement intéressant est la résistance aux maladies virales. Des plantes génétiquement transformées résistantes à différents virus ont été obtenues (Powell Abel et al 1986) (Cuozzo et al 1988) (Tumer et al 1987) (Hoekema et al 1989) (Van Dun et Bol 1988). Ces plantes (tabac, pomme de terre et tomate) expriment un gène codant pour la protéine de capside du virus pour lequel elles sont résistantes. Le mécanisme de protection n'est pas encore démontré.

La méthode classique pour protéger des plantes contre des maladies virales, consiste à inoculer des plantes avec une souche atténuée de virus pour prevenir l'infection par des souches plus virulentes. Cette pratique, appelée protection croisée, a permis de réduire les pertes de rendements dues aux infections virales (Broadbent 1976) (Fernow 1967) (Costa et Miller 1980).

Pour appliquer les techniques de génie génétique, un système de régénération de plantes à partir de cellules individuelles ou d'explants, mis en culture, sera indispensable.

De telles méthodes ont été décrites récemment pour la régénération des Cucurbitaceaes non-transformés :

La régénération du concombre, (Cucumis sativus) après induction de bourgeons adventifs sur des cals issus de cotylédons, a été décrite (Msikita et al, 1988; Kim et al, 1988); Wehner et Locy (1981) avaient déjà décrit l'induction de bourgeons sur des cotylédons. Des plantes de concombre ont pu être régénérées par embryogenèse somatique. Ces embryons somatiques se sont développés, soit en suspensions cellulaires issues de cals développés à partir d'explants foliaires (Chee and Tricoli, 1988) ou d'hypocotyles (Rajasekaran et al, 1983), soit directement sur des cals cotylédonnaires (Cade et al, 1988) ou foliaires (Malepszy and Nadolska-Orczyk, 1983). Dans tous les cas décrits ci-dessus le matériel nécessite le passage par une phase de callogenèse et de dédifférenciation cellulaire. Un passage prolongé en callogenèse peut induire des variations somaclonales non désirées. Dans certains cas, ces variations peuvent provoquer une stérilité chez les plantes

2

régénérées.

Pour le melon (C. melo), la régénération par organogenèse à déjà été décrite soit, directement sur des cotylédons mis en culture (Smith et al, 1988 ; Niedz et al, in press ; Dirks and Van Buggenum, 1989), soit après un passage par cals issus de cotylédons (Mackay et al, 1988 ; Moreno et al, 1985 ; Orts et al, 1987 ; Bouabdallah and Branchard, 1986), d'hypocotyles (Abak et Dumas de Vaulx, 1980 ; Kathal et al, 1986) ou de feuilles (Kathal et al, 1988).

L'obtention de plantes de melon issues d'embryons somatiques a également été rapportée (Oridate et Oosawa, 1986 ; Branchard et Chateau, 1988).

Toutes ces techniques nécessitent un passage par une étape plus ou moins longue de dédifférenciation et de callogènèse qui précéderont la différenciation de bourgeons ou d'embryons. Ces bourgeons peuvent se développer et donner soit des plantes ayant un phénotype anormal soit des plantes stériles (Bouabdallah et Branchard, 1986). De plus l'induction d'embryons ou de bourgeons issus de cals est plus faible comparé à l'induction directe de bourgeons sur des cotylédons.

Une technique de régénération a aussi été décrite chez une autre espèce de Cucurbitaceae<u>s</u> : La courge (Cucurbita pepo) (Jelaska, 1972, 1974). Cet auteur a obtenu des plantes à partir d'embryons somatiques issus de cals cultivés pendant plusieurs mois.

En ce qui concerne la régénération de plantes génétiquement transformées, EP-A-0262972 décrit la transformation de Cucumis sativus (concombre) par Agrobacterium rhizogenes suivie de régénération et EP-A-0265100 décrit la transformation de Cucumis sativus par fusion de protoplastes, suivie de régénération.

De Both et Ben Tahar (1989) ont rapporté l'obtention de cals de melon transformés. Ces cals qui se développent sur kanamycine et expriment le gène de la β-glucuronidase n'ont pas pu développer des plantes transgéniques, malgré l'application d'un protocole expérimental déjà utilisé avec succès dans la régénération de melon non-transformé.

EP-A-0257993 décrit la production de cals et de bourgeons transformés de <u>Cucumis melo</u>. En particulier, l'exemple IX décrit la transformation d'explants de <u>Cucumis melo</u> avec <u>Agrobacterium tumefaciens</u> contenant le gène <u>SURB-Hra</u> qui code pour une protéine "AL", résistante à certains herbicides. Les explants sont des cotylédons âgés de 7 à 14 jours. Après l'étape de transformation, les explants sont cultivés sur un milieu contenant les macro-éléments de Murashige et Skoog, additionné de 6 mgl$^{-1}$ de kinétine et de 1,5 mgl$^{-1}$ d'acide indole-3-acétique, pour donner des cals morphogéniques ou éventuellement des bourgeons. Les cals sont ensuite transférés sur un milieu dit "de régénération" contenant 0,1 mgl$^{-1}$ BAP. Les bourgeons obtenus sont ensuite transférés sur un milieu d'enracinement. L'obtention de plantules et de plantes transgéniques n'est pas décrite.

Le fait qu'il n'existe pas a ce jour de méthode de régénération à partir de tissus transformés chez l'espèce Cucumis melo, empeche d'obtenir des plantes transgéniques de melon exprimant des caractères agronomiquement intéressants tel que la résistance a des maladies virales.

Un des objectifs de l'invention est d'obtenir la régénération de plantes transgéniques appartenant a l'espèce Cucumis melo.

Un autre objectif de l'invention est de définir les milieux de culture qui sont nécessaires à chaque étape de la régénération de plantes transgéniques appartenant au genre Cucumis.

Cette invention s'applique plus particulièrement à la transformation génétique de cotylédons, d'hypocotyles, de feuilles de l'espèce Cucumis melo par l'utilisation d'Agrobacterium tumefaciens suivie de l'induction de bourgeons et de l'obtention de plantes génétiquement transformées. La transformation génétique des différents tissus, décrits ci-dessus, suivie de l'organogenèse permet l'amélioration des espèces cultivées et plus particulièrement de l'espèce Cucumis melo. Les plantes régénérées dans les conditions décrites dans cette invention ont un phénotype normal et sont fertiles.

Un autre objectif de cette invention est de transformer génétiquement le melon pour introduire un gène de résistance au virus de la mosaïque du concombre (CMV).

Ces objectifs sont atteints par le procédé de l'invention.

L'invention concerne un procédé de production de plantules transgéniques ayant un phenotype normal à partir de explants génétiquement transformés, lesdites plantules appartenant à l'espèce Cucumis <u>melo</u> et contenant au moins un gène introduit par l'intermédiaire d'Agrobacterium tumefaciens caractérisé par l'induction de bourgeons génétiquement transformés à partir de cotylédons de Cucumis melo ayant germé pendant 0 à 4 jours et ayant été mis à la suite de ce délai en contact avec Agrobacterium tumefaciens, suivie par la culture en deux étapes successives de bourgeons génétiquement transformés, la première de ces étapes de culture ayant lieu sur un milieu de culture cellulaire végétale contenant une cytokinine, et plus particulièrement la 6-benzyl aminopurine (BAP), et la deuxième, qui s'effectue lorsque les bourgeons ont atteint au moins environ 3 mm de hauteur, ayant lieu sur un milieu de culture cellulaire végétale contenant comme macro-éléments :

| $KH_2PO_4$ | environ | 50 à | environ | 100 | mgL-1 |
|---|---|---|---|---|---|
| $MgSO_4$ | " | 75 à | " | 300 | " |
| $CaCl_2.2H_2O$ | " | 500 à | " | 2500 | " |
| $KNO_3$ | " | 750 à | " | 1200 | " |
| $NH_4NO_3$ | " | 150 à | " | 200 | " |

L'invention concerne également un procédé d'induction de bourgeons génétiquement transformés à partir d'explants génétiquement transformés, et contenant au moins un gène introduit par Agrobacterium tumefaciens, lesdits explants provenant d'une plante de l'espèce Cucumis melo, l'induction s'effectuant sur un milieu d'induction de bourgeons génétiquement transformés comportant l'ensemble des sels minéraux et vitamines normalement requis pour l'induction de bourgeons à partir d'explants génétiquement non-transformés et contenant parmi les sels minéraux du chlorure de calcium et du bacto-agar ou agar-agar, caractérisé en ce que la teneur de ce milieu en $CaCl_2$ est d'environ 440 à environ 2200 mgL$^{-1}$ calculé en $CaCl_2.2H_2O$ et en bacto-agar ou en agar-agar est d'environ 0,8 à environ 1,2 %, ledit milieu d'induction étant additionné d'environ 0,3 à environ 1,13 mgL$^{-1}$ 6-benzyl aminopurine (BAP) et 0 à environ 1,3 mgL$^{-1}$ acide indole-3-acetic (AIA).

Selon un mode de réalisation préféré de l'invention, ce procédé d'induction de bourgeons transformés s'effectue sur un milieu d'induction dont la teneur en calcium est d'environ 1000 à environ 2200 mgL$^{-1}$, et plus particulièrement d'environ 1750 à environ 2200 mgL$^{-1}$.

En effectuant les procédés de l'invention, un explant d'une plante appartenant à l'espèce Cucumis melo peut être transformé et régénéré en plantes transgéniques. L'invention concerne également les plantes transgéniques, les plantules transgéniques, les bourgeons transformés et les explants transformés susceptibles d'être produits par les procédés de l'invention, ainsi que les semences.

La régénération de plantes transformées ou non-transformées à partir d'explants implique plusieurs étapes de culture cellulaire, chaque etape nécessitant un milieu de culture, des additifs, par exemple des cytokinines, et des conditions de culture très précises et qui varient souvent selon l'espèce à régénérer et selon la 'voie' de régénération (c'est-à-dire par organogenèse ou par embryogenèse somatique par exemple).

Même si un ensemble de milieux et de conditions est connu pour la régénération d'une plante non-transformée, il est impossible de prévoir si ces mêmes milieux et conditions pourront être appliqués avec succès dans la régénération de la plante lorsque celle-ci est dans l'état transformé. L'étape supplémentaire de transformation nécessite l'utilisation d'autres milieux, tels que des milieux de transformation et de co-culture, et d'autres additifs, par exemple, la cefotaxine, qui peuvent exercer un effet sur le comportement des cellules dans des milieux et conditions applicables dans les étapes ultérieures. La susceptibilité des plantes transformées à la vitrification est également un élément qui nécessite parfois le développement de conditions spéciales.

Des plantes vitrifiées sont caractérisées par une gamme d'anomalies morphologiques et physiologiques résultant des conditions de culture in vitro. Les causes souvent citées pour expliquer la vitrification, sont :
- Une concentration trop élevée en ions $NH_4$+ ou en cytokinines
- une concentration trop basse en agar (ou autre produit gélifiant) dans le milieu
- une sensibilité à l'éthylène produite par la plante et qui se trouve dans le volume du milieu de culture (Kevers et al, 1984).

La vitrification des cultures de plantules de melon in vitro due à la présence de cytokinines dans le milieu a été rapportée par Leshem et al (1984).

Tous ces éléments rendent donc la mise au point d'un ensemble de milieux et de conditions qui conduit à la régênération d'une plante transgénique extrêmement complexe.

Les inventeurs ont réussi à formuler un milieu d'induction de bourgeons qui permet l'induction de bourgeons génétiquement transformés à partir d'explants transformés. Ce milieu est un milieu d'induction de bourgeons comportant l'ensemble des sels minéraux et vitamines normalement requis pour l'induction de bourgeons à partir d'explants génétiquement non-transformés et contenant parmi les sels minéraux du chlorure de calcium et du bacto-agar ou agar-agar, la teneur de ce milieu en $CaCl_2$ étant d'environ 440 à environ 2200 mgL$^{-1}$ calculé en $CaCl_2.2H_2O$ et en bacto-agar ou en agar-agar étant d'environ 0,8 à environ 1,2 %. Ce milieu d'induction est additionné d'environ 0,3 à environ 1,13 mgL$^{-1}$ 6-benzyl aminopurine (BAP) et environ 0 à environ 1,3 mgL$^{-1}$ acide indole-3-acetic (AIA).

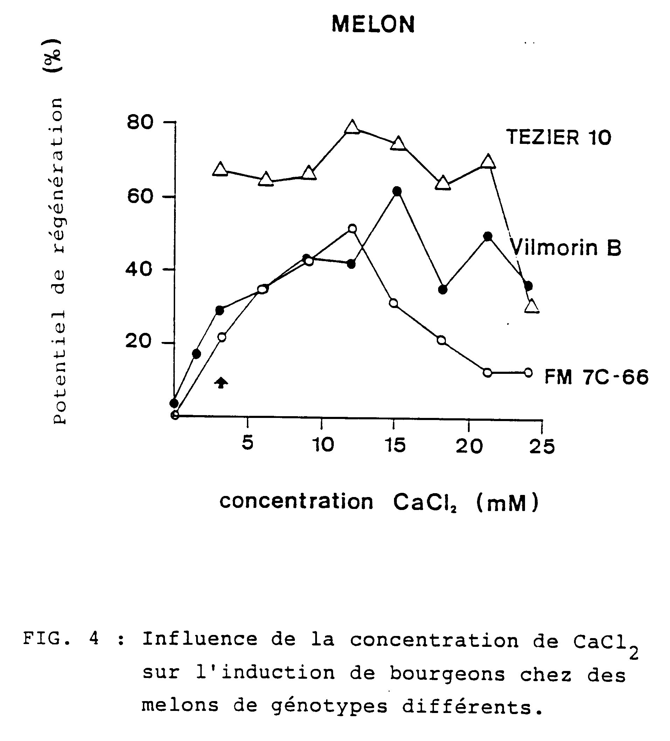

Un milieu d'induction particulièrement préféré est un milieu d'induction dont la teneur en calcium est d'environ 1000 à environ 2200 mgL$^{-1}$, et plus particulièrement d'environ 1750 à environ 2200 mgL$^{-1}$. Ces concentrations de calcium représentent une augmentation d'environ quatre à cinq fois les concentrations utilisées habituellement dans les milieux d'induction. D'une manière générale, les inventeurs ont constaté que des concentrations élevées de calcium ont un effet très bénéfique sur les étapes de régénération de melon transformé. La figure 4 montre l'effet de différentes concentrations de calcium sur l'induction de bourgeons chez différents génotypes de melon.

Selon un mode de réalisation de l'invention, le milieu d'induction de bourgeons peut être le milieu de Murashige et Skoog (1962), connu sous le nom milieu MS, dont la teneur en $CaCl_2$ et bacto-agar ou agar-agar est modifié, le cas échéant, en respectant les limites précisées ci-dessus. Le milieu MS, sans modification, a la composition suivante :

| Milieu MS | $(mgL^{-1})$ |
|---|---|
| $(NH_4)NO_3$ | 1,650 |
| $KNO_3$ | 1,900 |
| $CaCl_2.2H_2O$ | 440 |
| $MgSO_4.7H_2O$ | 370 |
| $KH_2PO_4$ | 170 |
| $FeSO_4.7H_2O$ | 27.8 |
| $Na_2EDTA$ | 33.6 |
| $MnSO_4.4H_2O$ | 22.3 |
| $ZnSO_4.7H_2O$ | 8.6 |
| $H_3BO_3$ | 6.2 |
| KI | 0.83 |
| $Na_2MoO_4.2H_2O$ | 0.25 |
| $CuSO_4.5H_2O$ | 0.025 |
| $CoCl_2.6H_2O$ | 0.025 |
| myo-inositol | 100 |
| acide nicotinique | 0.5 |
| pyridoxine.HCl | 0.5 |
| thiamine.HCl | 0.1 |
| glycine | 2.0 |
| saccharose | 0,000 |
| pH | 5.7 |

Le milieu MS solidifié contient en outre 0.8 % agar-agar ou bacto-agar.

Un milieu d'induction préféré est le milieu MS, additionné de 0.3 à 1.13 mgL-1 BAP, et plus particulièrement 0.85 mg/L BAP en l'absence de toute autre cytokinine.

Les vitamines présentes dans le milieu d'induction peuvent être celles présentes normalement dans le milieu MS. Selon un autre mode de réalisation de l'invention, ces vitamines peuvent être les vitamines 'Staba' (Staba, 1969)

## Vitamines de "Staba"

| | | |
|---|---|---|
| nicotinamide | 2 | $mgL^{-1}$ |
| pyridoxine-HCl | 2 | $mgL^{-1}$ |
| d-biotine | 1 | $mgL^{-1}$ |
| Ca-panthothenate | 1 | $mgL^{-1}$ |
| thiamine-HCl | 1 | $mgL^{-1}$ |
| chlorure de choline | 1 | $mgL^{-1}$ |
| acide p-amino benzoique | 0,5 | $mgL^{-1}$ |
| acide folique | 0,5 | $mgL^{-1}$ |
| riboflavine | 0,5 | $mgL^{-1}$ |
| cianocobalamine | 1,5 | $\mu gL^{-1}$ |

Un milieu d'induction de bourgeons particulièrement préféré est le milieu appelé "milieu M.I." mis au point par les inventeurs. Le milieu M.I. a la composition suivant :

## Milieu M.I.

| | | | |
|---|---|---|---|
| Macro-éléments : | $KNO_3$ | 1900 | $mgL^{-1}$ |
| | $NH_4NO_3$ | 1650 | |
| | $CaCl_2.2H_2O$ | 2200 | |
| | $MgSO_4.7H_2O$ | 370 | |
| | $KH_2PO_4$ | 170 | |
| $NA_2EDTA$ | idem MS | | |
| Micro-éléments | idem MS | | |
| Vitamines | Staba | | |
| Myo-inositol | 100 | $mgL^{-1}$ | |
| Saccharose | 30 | $gL^{-1}$ | |
| Agar-agar | 0.8 % | | |
| BAP | 3.75 $\mu M$ | | |
| ABA | 1 $\mu M$ | | |

La teneur en Agar du milieu M.I. solidifié peut être modifié de 0.8 % à 1 % (p/v). 0.8 % est préféré. Il est possible de rajouter environ 1 µM d'acide abscissique aux milieux d'induction selon l'invention.

Un autre milieu mis au point par les inventeurs est celui qui permet l'obtention de plantules transgéniques à partir de bourgeons transformés.

Ce milieu est un milieu de culture cellulaire végétale qui contient comme macro-éléments minéraux :

| $KH_2PO_4$ | environ 50 | – | environ | 100 | $mgL^{-1}$ |
|---|---|---|---|---|---|
| $MgSO_4.7H_2O$ | " 75 | – | " | 300 | " |
| $CaCl_2.2H_2O$ | " 500 | – | " | 2500 | " |
| $KNO_3$ | " 750 | – | " | 1200 | " |
| $NH_4NO_3$ | " 150 | – | " | 200 | " |

Les autres constituants du milieu, par exemple, micro-éléments, vitamines, etc... sont ceux normalement utilisés dans les milieux de culture cellulaire, par exemple le milieu MS, et dans les concentrations habituelles. La teneur en saccharose peut être réduit jusqu'à environ 10 $gL^{-1}$.

Dans un mode de réalisation préféré de l'invention, le milieu pour le développement de plantules transgéniques contient comme macro-éléments :

$KH_2PO_4$ 85 mg $L^{-1}$
$MgSO_4.7H_2O$ 185 $mgL^{-1}$
$CaCl_2.2H_2O$ 1720 $mgL^{-1}$
$KNO_3$ 950 $mgL^{-1}$
$NH_4NO_3$ 165 $mgL^{-1}$

Il est à noter que la teneur en $CaCl_2.2H_2O$ selon cet aspect de l'invention est d'environ cinq fois plus élevé que dans des milieux classiques. Par contre, la concentration de $KH_2PO_4$, de $MgSO_4$ $7H_2O$ et de $KNO_3$ est d'environ la moitié de ce qu'on trouve dans d'autres milieux de ce type, et celle de $NH_4NO_3$ est d'environ un dixième.

Un milieu particulièrement préféré de l'invention pour le développement des plantules transgéniques, est le milieu appelé MB6, dont la composition est indiquée dans le Tableau 1.

Ce milieu, mis au point par les inventeurs est essentiel pour obtenir des plantules transgéniques à partir de bourgeons. Cependant, il est important que la culture des bourgeons soit effectuée en deux étapes, la première de ces étapes ayant lieu sur un premier milieu de culture cellulaire végétale contenant une cytokinine. La cytokinine peut être la kinetine ou la BAP. La BAP est particulièrement préférée. Un premier milieu de culture cellulaire approprié est, par exemple le milieu MS additionné de 0.1 - 1.2 $mgL^{-1}$ BAP et en particulier d'environ 0.2 $mgL^{-1}$. Ce milieu de culture est normalement solidifié par l'addition d'environ 0.8 % agar agar ou bacto agar.

Lorsque les bourgeons ont atteint environ 3 mm de hauteur ou plus sur ce premier milieu, ils sont transférés sur un deuxième milieu qui est le milieu permettant l'obtention des plantules transgéniques et défini en détail ci-dessus, par exemple le milieu MB6. Ce milieu sera appelé dans ce qui suit 'le milieu du type MB6'.

Sur ce milieu du type MB6, les bourgeons d'au moins 3 mm de hauteur s'allongent et au bout d'environ 4 semaines, les plantules s'enracinent. Une fois que les racines secondaires se sont développées sur ce milieu, les plantes peuvent être transférées dans des pots contenant un mélange de terreau et gros sable et mises en culture à la serre. Les plantes transgéniques ainsi obtenues ont un phénotype normal et sont fertiles.

Bien entendu, les milieux de l'invention sont additionnés, le cas échéant, d'agents permettant la sélection de transformants, par exemple des antibiotiques, et d'agents qui inhibent la croissance de A.tumefaciens, par exemple la cefotaxime. Les concentrations de cefotaxime pouvant être utilisées sont de l'ordre d'environ 200 $mgL^{-1}$ à environ 400 $mgL^{-1}$. L'application simultanée d'une sélection avec de la kanamycine permet d'utiliser environ 200 $mgL^{-1}$ de cefotaxime pour l'inhibition. Il est particulièrement préféré d'utiliser 200 $mgL^{-1}$ de cefotaxime puisque le développement des plantules n'est pas affecté par cette concentration plutôt faible. Les concentrations de kanamycine utilisées peuvent varier entre environ 50 à 400 $mgL^{-1}$. Si le milieu M.I. est utilisé comme milieu de sélection, la concentration de kanamycine doit être de 150 $mgL^{-1}$ à 400 $mgL^{-1}$.

Selon un mode de réalisation de l'invention, le milieu d'induction de bourgeons et le milieu du type MB6 peuvent être utilisés de manière successive dans la régénération de plantes transgéniques de melon à partir d'explants transformés. Les bourgeons induits par la culture sur le milieu d'induction sont excisés de l'explant et sont transférés sur le premier milieu de développement de plantules contenant de la cytokinine, par exemple la BAP, et ensuite sur le milieu du type MB6.

Selon un autre mode de réalisation de l'invention, des explants de plantes appartenant à l'espèce Cucumis melo, sont transformés par Agrobacterium tumefaciens et les explants transformés ainsi obtenus sont ensuite cultivés sur le milieu d'induction de bourgeons. Les bourgeons sont alors soumis à l'étape de développement de plantules transgéniques, qui, comme déjà expliqué, se déroule en deux étapes, la dernière de ces étapes étant effectuée sur le milieu du type MB6. Finalement, les plantules transgéniques ainsi obtenus sont cultivées par des méthodes déjà connues dans le développement de plantules non-transformés, pour obtenir des plantes transgéniques.

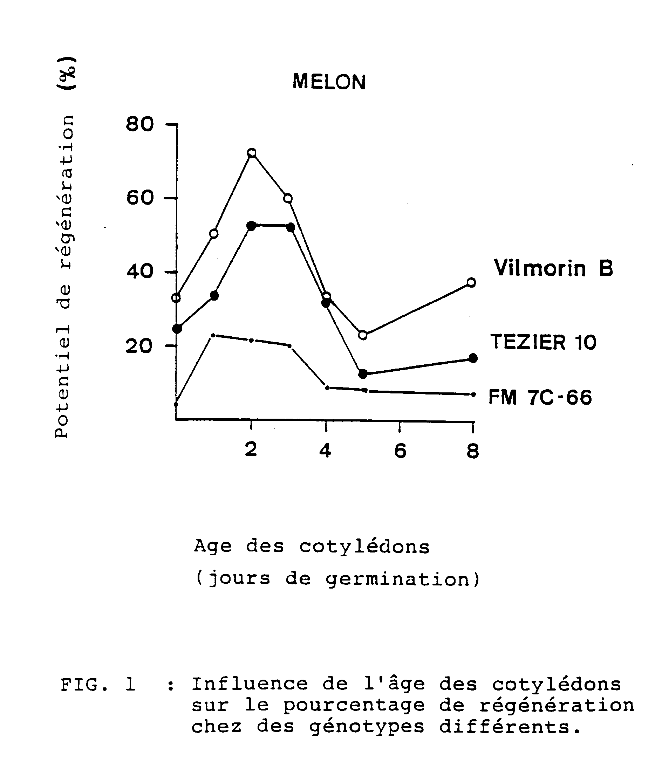

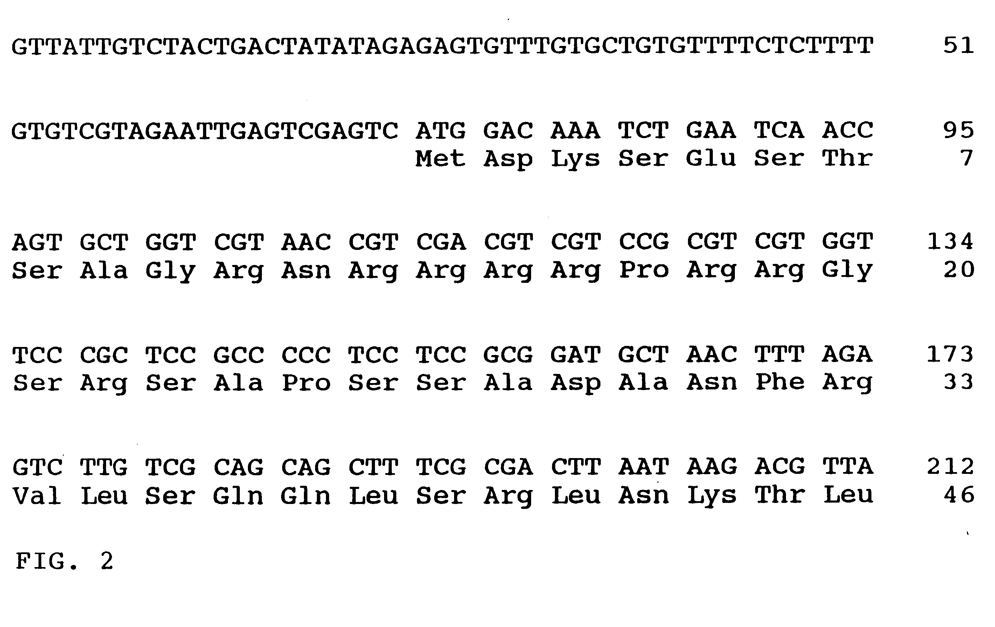

L'explant utilisé comme matériel de départ pour la transformation et la régénération de melons transgéniques peut être n'importe quel explant transformable par Agrobacterium tumefaciens, par exemple : cotylédons, hypocotyles, feuilles. L'utilisation de cotylédons comme explant est particulièrement avantageuse. Les inventeurs ont constaté que le potentiel de régénération de bourgeons sur des cotylédons prélevés d'embryons ayant jusqu'à quatre jours de culture, et plus particulièrement deux jours de culture est supérieur à celui présenté par des cotylédons plus jeunes ou plus anciens (voir Fig 1). Ce phénomène a été observé chez de nombreuses variétés différentes. Il est important de préciser que les cotylédons sont issus d'embryons qui ont germé pendant 0 à 4 jours, les embryons étant isolés de graines matures de melon. Une germination à partir de graines ne conduit pas aux mêmes résultats avantageux. Le milieu de germination est de préférence 0.8 % (poids/volume) d'agar-agar dans de l'eau stérile, qui peut être additionné de 50 $\mu$m $CoCl_2$ ou $NiCl_2$, ce qui semble améliorer l'induction des bourgeons dans l'étape suivante. Comme indiqué plus haut, la durée de la germination est de 0 à 4 jours. Une durée de moins de trois jours et notamment de deux jours, est particulièrement préférée. La germination s'effectue normalement sous une intensité lumineuse de 60 à 80 $\mu Em^{-2}s^{-1}$, et une photopériode de 16/24 heures à environ 26°C le jour et 24°C la nuit.

La transformation génétique de l'explant de melon s'effectue par la mise en contact d'une souche de A.tumefaciens, par exemple sous forme de suspension bactérienne, et l'explant qui a subi une blessure, par exemple par coupure. Dans le cas de cotylédons, la mise en contact avec les bactéries est effectuée lorsque les cotylédons sont âgés de 0 à 4 jours, et plus particulièrement de 2 jours.

La durée de cette mise en contact n'est pas critique; par exemple, elle peut durer environ 30 minutes à une heure, et s'effectue par exemple dans une suspension dans un milieu de culture M.S.

Les explants sont alors séchés sur papier filtre stérile, et transférés sur un milieu de co-culture, qui peut être du milieu MS solidifié avec de l'agar-agar. Les conditions d'intensité lumineuse et de photopériode qui sont appliquées lors de la co-culture sont en général les mêmes que celles utilisées lors de l'étape de germination. Normalement, cette étape de co-culture dure environ 48 heures.

De nombreuses souches à nopaline et octopine de A. tumefaciens sont connues et peuvent être utilisées pour la transformation génétique de l'espèce Cucumis melo.

Les inventeurs ont utilisé la souche à octopine LBA4404 contenant le plasmide Ti pAL4404 plus le vecteur binaire pGA472 offert par G. Ann, Washington University (Ann et al, 1985) ou la souche à nopaline C58'3 (Ben Tahar et De Both, 1988). Ces souches ont été curées de leur vecteur et le vecteur binaire pBI121 (offert par R. Jefferson, Plant Breeding Institut) (Jefferson et al, 1987) a été introduit dans la souche LBA4404 ne contenant pas le vecteur pGA472. Ce vecteur binaire possède un gène marqueur de résistance à la kanamycine et un gène "reporter", le gène de la $\beta$-glucuronidase codant pour une activité enzymatique. Ce gène de résistance à la kanamycine contient la région codante du gène de la néomycine phosphotransférase isolé du transposon Tn5 flanquée en 5' et 3' par le promoteur et terminateur du gène de la nopaline synthase. La région codante du gène de la $\beta$-glucuronidase est flanquée du promoteur de l'ARN 35S du virus de la mosaïque de chou fleur et du terminateur du gène de la nopaline synthase.

La souche LBA4404 contenant le vecteur pBI121 est conservée à - 20°C dans 15 % glycérol. Cette souche pousse sur un milieu LB contenant 50 mg/L kanamycine.

Après l'étape de co-culture, les explants transformés sont transférés sur le milieu d'induction de bourgeons décrit dans les paragraphes précédents. Ce milieu est normalement additionné d'agents permettant la sélection des transformants, par exemple la kanamycine, et d'agents inhibant la croissance de A.tumefaciens, par exemple la cefotaxime.

Les inventeurs ont constaté que l'utilisation de boîtes de Petri "Falcon"™ de 9 cm de diamètre donne des résultats particulièrement avantageux lors de l'étape d'induction de bourgeons. Les conditions d'intensité lumineuse et de photopériode qui s'appliquent pendant cette etape sont les mêmes que celles appliquées lors de l'étape de germination, une intensité lumineuse de 80 $\mu E.M^{-2}s^{-1}$ étant particulièrement préférée.

Des bourgeons se développent directement sur l'explant et lorsqu'ils ont atteint une taille qui permet leur manipulation physique (environ 0.2 - 1.5 mm, et plus particulièrement 0.5 mm), ils sont excisés de l'explant et transférés sur un nouveau milieu, qui normalement est celui qui contient de la BAP ou une autre cytokinine et qui constitue le 'premier' milieu de culture pour le développement de plantules.

Dans une variante du procédé selon l'invention, la durée de l'étape d'induction, qui normalement est d'environ 21 jours, peut être raccourcie à 14 jours. Dans ce cas les bourgeons, au lieu d'être transférés directement sur le premier milieu de développement de plantules peuvent être transférés sur un milieu MS sans hormones pour une semaine avant d'être soumis à l'étape de développement de plantules.

L'étape de développement de plantules se déroule en deux parties comme expliqué plus haut. Les bourgeons s'allongent sur ce milieu et lorsqu'ils ont atteint un hauteur d'environ 3 mm à 1.0 cm, ils sont transférés sur le milieu MB6, additionné d'agents sélectifs pour sélectionner les transformants, par exemple des antibiotiques et d'agents qui inhibent la croissance de A. tumefaciens. Néanmoins, ces agents ne sont pas essentiels dans la régénération.

Au bout de quelques semaines sur ce milieu, les plantules transgéniques s'enracinent. Les plantes peuvent être transférées dans des pots contenant du terreau et du gros sable (2:1 v/v) aussitôt que les racines secondaires se sont développées.

Selon un mode de réalisation préféré de l'invention, un gène codant pour la protéine de capside du virus de la mosaïque de concombre est introduit dans l'explant de melon par l'intermédiaire de A. tumefaciens.

Ce virus est composé de 4 ARNs de taille différentes ; le gène de la protéine de capside du CMV est présent sur l'ARN 3 à l'extrémité 3' et sur l'ARN 4. La traduction en protéine de capside ne se fait qu'à partir de l'ARN4, l'extrémité 3' de l'ARN 3 a strictement la même séquence nucléotidique. Le gène de la protéine de capside de deux souches virulentes provenant de différentes régions géographique, a été isolé et séquencé par les inventeurs.

Dans cet aspect de l'invention une souche de CMV virulente française, appelée la I17F, et un plasmide (pUC18) contenant l'ADN complémentaire à l'ARN 3 d'une souche virulente, la FNY, isolée à New York sur des melons infectés, ont été utilises. Il va de soi que n'importe quel autre gène pourrait ainsi être introduit dans les explants de melon, par exemple des gènes codant pour la protéine de capside d'autres souches de CMV, et d'autres virus, le procédé de l'invention permettant alors la régénération de la plante transgénique contenant ce gène.

**Exemples**

**A) Procédé de production de melon transgénique exprimant le gène de résistance à la kanamycine et le gène de la β-glucuronidase**

1. GERMINATION DES EMBRYONS DE CUCUMIS MELO

Des graines matures de melon (Cucumis melo L) sont utilisées pour l'obtention d'embryons matures. Les téguments de la graine sont ouverts au scalpel évitant de blesser les embryons. Les embryons sont stérilisés par immersion pendant 5 secondes dans de l'éthanol pur puis dans 200 ml d'une solution saturée d'hypochlorite de calcium et 0,05 % de Tween 80 pendant 20 minutes sous agitation constante. Les embryons sont ensuite rincés dans 200 ml d'eau stérile pendant 20 minutes. Les embryons sont ensuite séchés sur papier filtre et déposés dans des boîtes de pétri stériles contenant 0.8 % (poids/volume) d'agar-agar dans de l'eau stérile. Les embryons sont mis en germination pendant deux jours dans des chambres de culture sous une intensité lumineuse de 60 à 80 $\mu Em^{-2} s^{-1}$ et une photopériode de 16/24 heures à 26°C le jour et 24°C la nuit.

2. LES SOUCHES D'AGROBACTERIUM TUMEFACIENS ET LES VECTEURS DE TRANSFORMATION

Pour les expériences de transformation la souche LBA4404 contenant le vecteur pBI121 (décrit dans les pages précédents) est mise en culture dans 10 ml du milieu Luria-Bertani sans antibiotique sous agitation continue (200 rpm) à 28°C pendant 12 heures. Cette solution bactérienne va servir d'innoculum à un plus grand volume de LB (milieu Luria-Bertani) (50 ml) supplémenté 100 mg $L^{-1}$ rifampicine et 50 mg $L^{-1}$ kanamycine. Le milieu LB est composé de 10 g bactotryptone, 5 g d'extraits de levure et de 10 g NaCl par litre à pH 7.5. Cette suspension bactérienne est maintenue sous agitation à 28°C pendant 4 à 5 heures jusqu'à ce que la densité optique soit de 1 à une longueur d'onde de 660 nanomètres. La suspension bactérienne est ensuite centrifugée à 4000 rpm pendant 5 minutes et le culot est repris dans le même volume initial dans du milieu MS. Cette solution est prête à être mise en contact avec le tissu végétal.

3. PROCEDURE DE TRANSFORMATION

Les cotylédons issus d'embryons qui ont germé pendant deux jours sur le milieu décrit dans le paragraphe 1, sont prélevés avec précaution afin d'éviter les tissus du méristème apical. Des cotylédons plus jeunes ou plus anciens ont un potentiel de régénération de bourgeon réduit comparé aux cotylédons prélevés d'embryons ayant deux jours de culture. Ce résultat est montré sur la figure (1).

Ces cotylédons de deux jours sont coupés en 4 dans la suspension bactérienne reprise dans le milieu MS afin d'assurer, après la blessure, un contact avec la souche LBA4404 contenant le vecteur binaire pBI121. Les fragments de cotylédons sont laissés 30 minutes à une heure dans la suspension bactérienne. Chaque fragment est séché sur papier filtre stérile et transféré dans des boîtes de pétri contenant du milieu MS solidifié avec 0.8 % d'agar-agar.

Les explants sont mis en culture dans une chambre de culture in vitro dans les conditions d'intensité lumineuse et de photopériode décrites dans le paragraphe 1 et pendant 48 heures.

## 4. INDUCTION DE BOURGEONS

Après cette coculture de deux jours décrite ci-dessus, les fragments de cotylédons sont transférés sur un nouveau milieu contenant du MS additionné de 400 mg.L$^{-1}$ cefotaxime (Roussel-Uclaf), 50 mg.L$^{-1}$ kanamycine sulfate, 1,13 mg.L$^{-1}$ BAP et 0,88 mg.L$^{-1}$ AIA. La présence de cefotaxime est requise pour inhiber la croissance de la bactérie. La kanamycine sulfate est utilisée comme agent sélectif pour sélectionner ainsi les cellules transformées. Les cellules transformées seront celles qui posséderont dans leur génome le gène de résistance à la kanamycine et le gène de la B glucuronidase décrit dans le paragraphe 2. L'hormone végétale BAP ajoutée au milieu de culture est nécessaire pour l'induction de bourgeons aux concentrations décrites ci-dessus. La présence de AIA est, par contre, facultatif.

Ces cellules ainsi transformées induiront des bourgeons qui se développeront sur 50 mg/l de kanamycine.

Les fragments de cotylédons sont incubés dans des chambres de culture in vitro dans les conditions d'intensité lumineuse et de photopériode décrites dans le paragraphe 1.

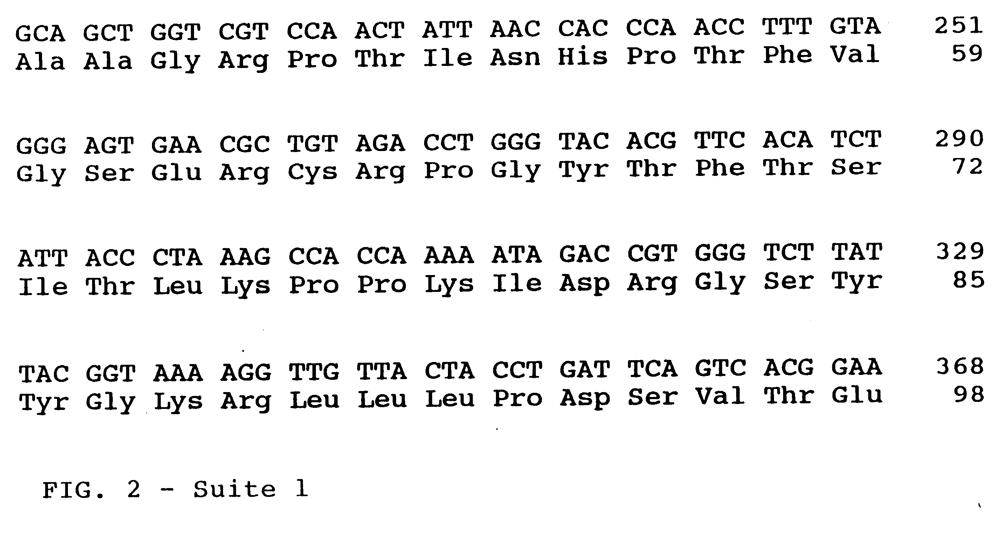

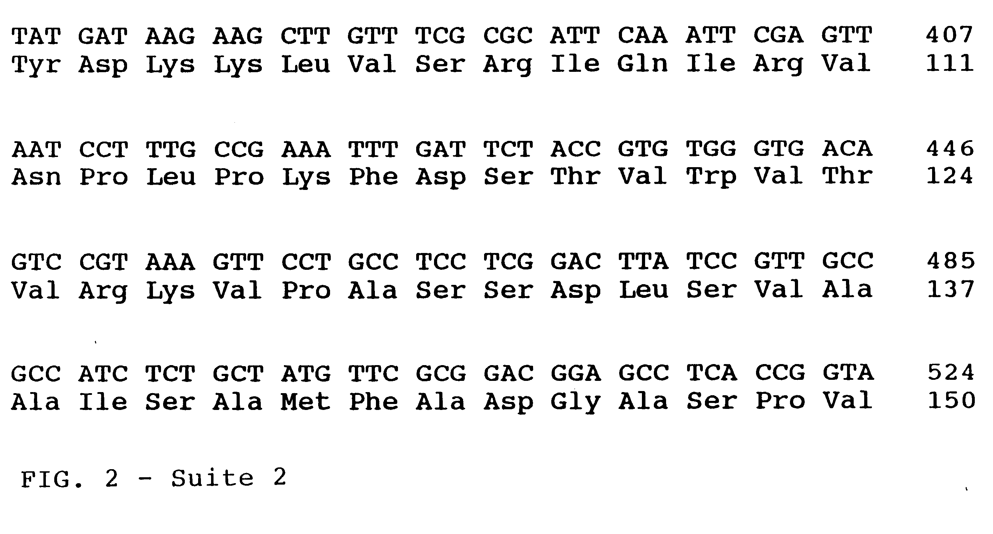

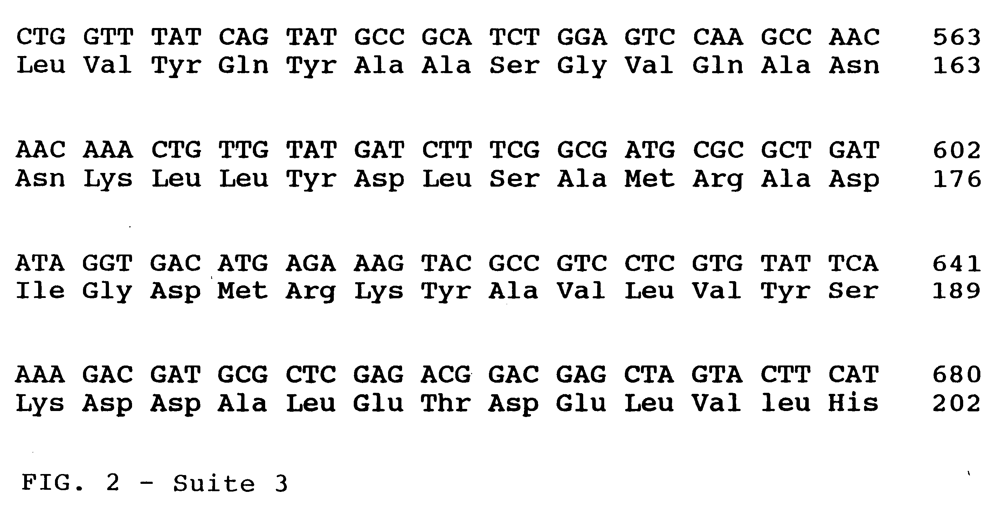

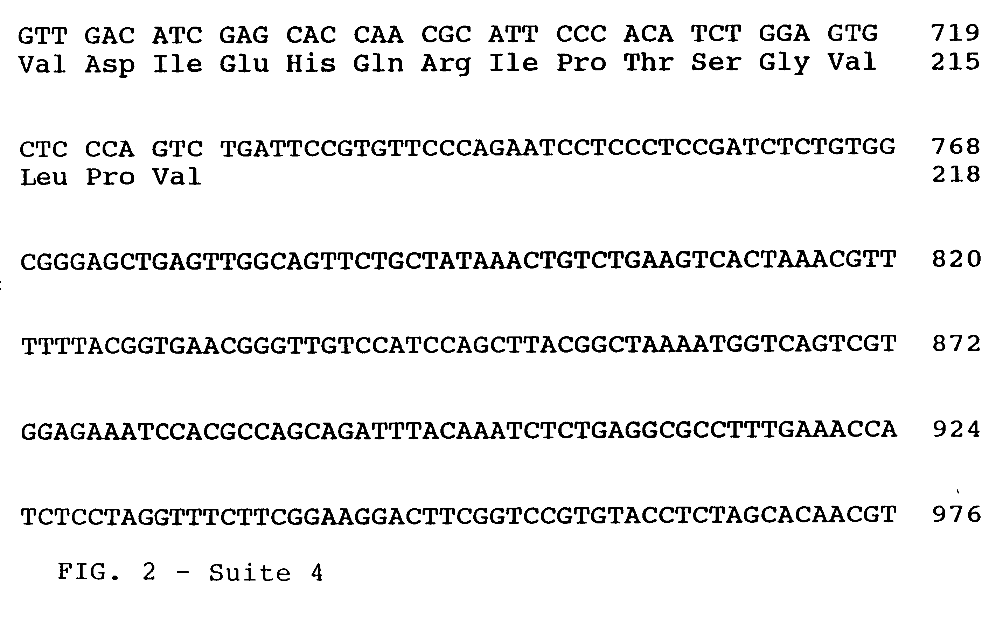

Au bout de 3 à 5 semaines des bourgeons se développent en présence de 50 mg.L$^{-1}$ de kanamycine directement à partir des fragments de cotylédon. Avec ce milieu d'induction, aucun bourgeon ne peut se développer sur 50 mgl$^{-1}$ kanamycine à partir de fragments de cotylédon non transformés. Cet effet est montré sur la figure 2.

## 5. DEVELOPPEMENT DE PLANTULES TRANSGENIQUES

Lorsque les bourgeons, induits directement sur les fragments de cotylédons cultivés sur le milieu décrit dans le paragraphe 3, en présence de 50 mgL$^{-1}$ de kanamycine, atteignent la taille de 0,5 mm de hauteur, ils sont excisés des fragments de cotylédons. Ces bourgeons sont transférés individuellement sur un nouveau milieu de culture contenant du MS additionné de 0,68 mg.L$^{-1}$ BAP, 400 mg.L$^{-1}$ cefotaxime et 50 mg.L$^{-1}$ kanamycine sulfate. Ces bourgeons s'allongent sur ce milieu et après 2 à 4 semaines lorsqu'ils atteignent 3 mm de hauteur, ils sont à nouveau transférés individuellement, cette fois dans des pots de culture de 500 ml de volume sur 100 ml de milieu MB6 (voir tableau 1) contenant 50 mg.L$^{-1}$ kanamycine sulfate et 400 mg.L$^{-1}$ cefotaxime.

Sur ce milieu décrit ci-dessus, les bourgeons de 3 mm de hauteur s'allongent et au bout de 4 semaines les plantules transgéniques s'enracinent. Une fois que les racines secondaires se sont développées sur le milieu décrit ci-dessus, les plantes sont transférées dans des pots contenant un mélange de terreau et gros sable (2:1) et mises en culture à la serre. Pendant les sept premiers jours, les plantes sont protégées par un couvercle en plastique afin de conserver une atmosphère humide. Les plantes sont arrosées journellement. La photo 1 présente une plante transgénique (B) et une plante non transgénique (A).

Une des plantes ainsi régénérées (appelée la 884-5) a été amenée à floraison et autofécondée. Les semences ont été recueillies. Quatre plantes R1 ont germiné et leur caractère transgénique a été confirmé en appliquant les tests décrits ci-dessous.

Le premier test, qui détecte l'activité de l'enzyme B-Glucuronidase (G.U.S) est effectué selon la méthode de Jefferson et al (1987). Un autre test implique l'induction de cals sur une feuille ou sur un explant de pétiole sur un milieu contenant de la kanamycine. La formation d'un cal indique que la plante est transgénique. La concentration de kanamycine nécessaire dans un milieu M.S. contenant de la BAP et de la NAA, varie en fonction de la concentration d'hormone et du tissu. Par exemple, dans un milieu M.S. additionné de 1 mgL$^{-1}$ de BAP et de NAA, 100 mgL$^{-1}$ de kanamycine suffisent pour inhiber la formation de cals sur des feuilles et sur des pétioles.

La présence des gènes étrangers chez les plantes régénérées a également été testée avec la réaction de polymérase en chaîne (P.C.R.) (voir Lassner et al 1989; De Both 1990) en utilisant 2.5 unités de Taq polymérase. Les amorces utilisées permettaient l'amplification simultanée du gène GUS et du gène NPT. La PCR n'a été effectuée que sur la génération R1, afin d'éviter des fausses positives dues à la présence d'Agrobacteria dans le tissu régénéré.

**B) Production de melons transgéniques exprimant un gène de résistance à l'herbicide phosphinotricine**

Le vecteur binaire pIB16.1 (Hoechst, GmbH, Frankfurt, FRG) comporte le gène neo et le gène pat (phosphinothricin acetyl transferase), qui confère la résistance à la phosphinotricine.

Le procédé de transformation et de régénération décrit dans l'exemple A a été utilisé dans la production de plantes de génotype Vedrantais, transformée par le vecteur. Le caractère transgénique des plantes ainsi obtenu a été confirmé par le test de formation de cals, et l'hybridation de sondes de neo selon la méthode de Southern.

Ces plantes ont été autofécondées et les semences ainsi obtenues germées sous serre. Les plantes ainsi obtenues ont subi un traitement avec l'herbicide et certaines se sont montrées résistantes.

## C) **Production de melons transgéniques exprimant la protéine de capside du virus de mosaïque de concombre (CMV)**

Dans les exemples qui suivent, une souche de CMV virulente française, appelée la I17F et un plasmide (pUC18) contenant l'ADN complémentaire à l'ARN3 d'une souche virulente, appelée la FNY, isolée à New York sur des melons infectés, ont servi comme source de gènes de protéines de capside de CMV.

## 1. CLONAGE DE LA PROTEINE DE CAPSIDE DE LA SOUCHE FNY

A partir de la carte de restriction du plasmide pUC18 contenant l'ADNc de l'ARN 3 déterminé par P. Palukaïtis un fragment d'une taille de 1.6Kb couvrant à l'extrémité 3' la séquence de la protéine de capside de 1Kb a été sous cloné dans un vecteur d'expression, "Blue scribe". Cet ADNc a été sous cloné dans le vecteur Blue Scribe (BS+) (catalogue Stratagène) et chaque fragment sous cloné a été séquencé suivant le protocole "séquenase R" (version 2 de USB: United State Biochemical). Les fragments séquencés ont été analysés sur gel d'acrylamide 6 % dénaturant tel décrit dans Maniatis et al 1982. Le gel est ensuite fixé 10 minutes dans 10 % acide acétique, séché sous vide pendant 30 minutes à 80°C puis mis en autoradiographie (Maniatis et al 1982). Le début de la séquence leader de la protéine de capside de la souche FNY a la même longueur que celle de la souche Y japonaise (communication privé avec P. Palukaïtis). La séquence partielle de l'ARN 4 de la souche Y a été publiée (Hidaka et al 1985). Par comparaison avec cette séquence nous avons pu positionner le début de la séquence leader de la souche FNY.

La séquence de la protéine de capside couvrant la séquence leader, la région codante et la région 3' non codante est représenté sur la Fig 2. A partir de cette séquence 2 oligonucléotides de 30 bases complémentaires en 3' et 5' ont été synthétisés et la séquence de la protéine de capside amplifiée en utilisant la technique PCR (Polymérase Chain Reaction) décrite par Scharf et al (1986). Le produit de l'amplification a été analysé sur gel d'agarose 1% pour confirmer la taille de ce fragment de 1034bp. Les oligonucléotides en 5' et 3' possèdent des sites de restriction BamH1 a leur extrémité. Ainsi ce produit d'amplification a pu être cloné au site unique BamH1 du vecteur pBIOS3. Ce vecteur pBIOS3 construit dans le laboratoire des inventeurs possède un promoteur et un terminateur végétal. Le promoteur 35S provenant de l'ARN 35S du virus de la mosaïque du choufleur et le terminateur NOS provenant de la région 3' non codante du gène de la nopaline synthase.

## 2) CLONAGE DE LA PROTEINE DE CAPSIDE DE LA SOUCHE I17F

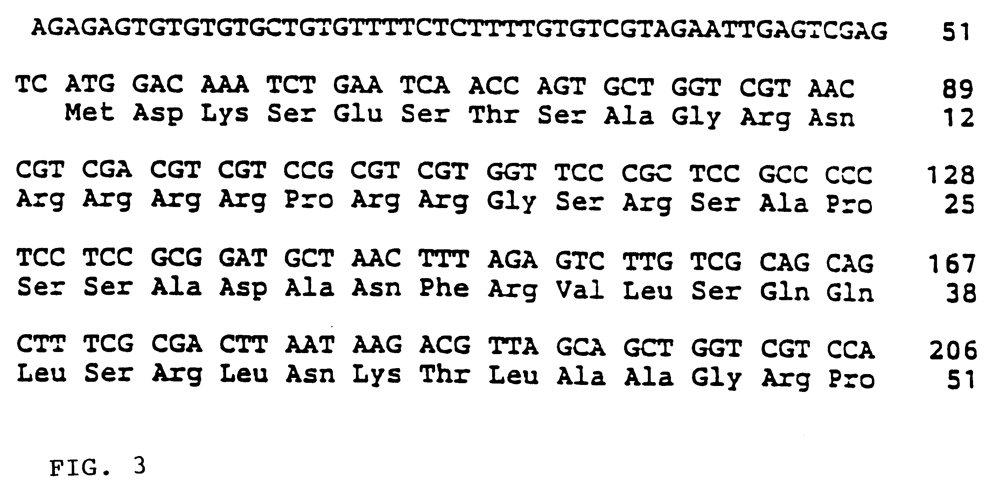

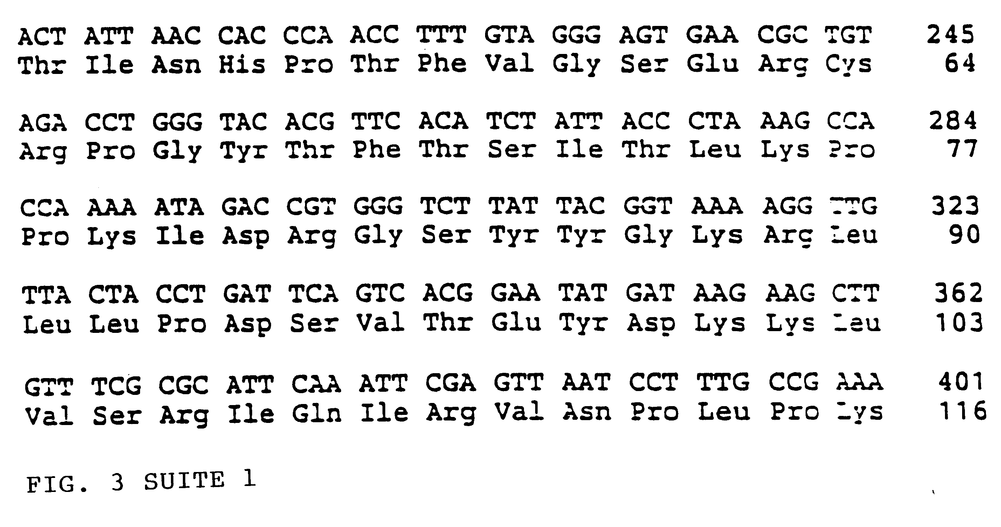

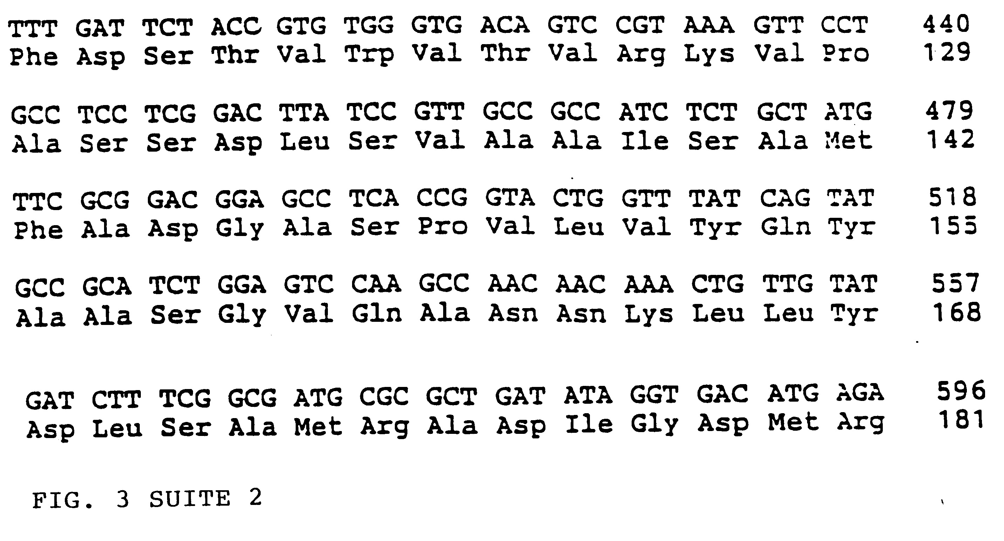

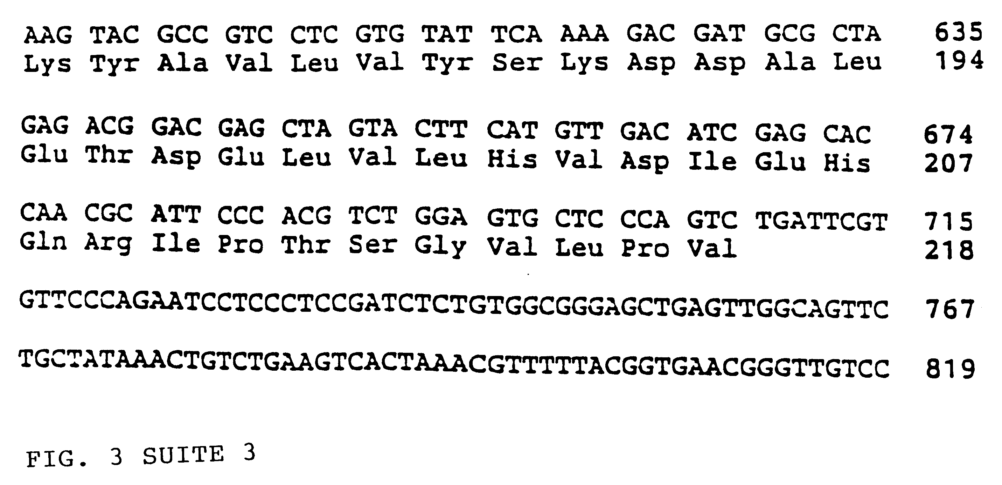

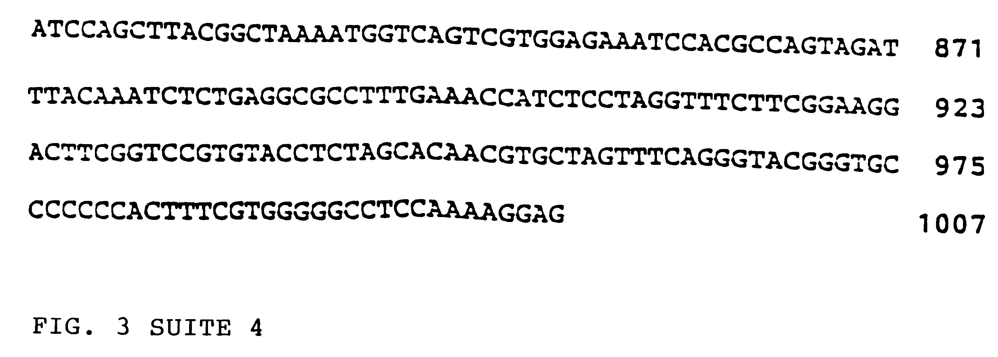

Des plantules de tomate au stade 2 feuilles ont été inoculées avec la souche I17F du CMV. 15 jours après l'infection, le virus a été purifié à partir des feuilles infectées selon la technique décrite dans la thèse du DR. G. Marchoux (1975). La qualité de l'ARN viral (ARN 1, 2, 3 et 4) est contrôlée sur gel d'agarose 1 %. Une fois contrôlé un ADN complémentaire aux 4 ARNs est synthétisé en utilisant le kit Amersham "cDNA Synthesis System plus". L'ADN radioactif complémentaire aux 4 ARNs est déposé sur un gel d'agarose 1%. Après migration de ces ADNs le gel est séché sous vide puis mis en autoradiographie (Maniatis et al 1982). Ces ADNs complémentaires possédant des extrémités à bout franc sont ensuite clonés au site Sma1 du plasmide pUC 18 déphosphorylé (Maniatis et al 1982). Ce plasmide porte la résistance à l'ampicilline. Des bactéries E. Coli JM101, rendues compétentes par la méthode au chlorure de calcium (Maniatis et al 1982), sont transformées par les plasmides pUC18 contenant les ADN complémentaires aux ARNs viraux. Le plasmide pUC 18 portant la résistance à l'ampicilline, les colonies bactériennes transformées sont sélectionnées sur le milieu Luria Broth (Maniatis et al 1982) additionné d'ampicilline à 100 mg/L d'une part et par la couleur bleue des colonies (Maniatis et al 1982) d'autre part. Le plasmide recombinant est isolé de chaque colonie et purifié selon la technique de Birnboin et Doly (Maniatis et al 1982). Les plasmides recombinants sont digérés aux enzymes de restriction EcoR1 et BamH1; ces enzymes coupant autour du site Sma1 permettent de séparer par électrophorèse le plasmide pUC 18 d'une part et l'ADN complémentaire d'autre part. Les produits de digestion sont déposés sur un gel d'agarose 1 % et après migration les plasmides contenant un ADN complémentaire d'une taille de 1kb (taille de l'ADN complémentaire) sont sélectionnés. Une carte de restriction en utilisant les enzymes HindIII, Sall et HindII a permis par comparaison avec celle de la souche D publiée par Cuozzo et al (1988) d'identifier les plasmides recombinants possédant l'ADN complémentaire à l'ARN 4. Ce clone possédant le gène codant pour la protéine de capside a été recloné entre les sites de restriction EcoRI et BamHI du plasmide Bluescribe (BS+ et BS-) (catalogue Stratagène). Des fragments de taille plus petite ont été recloné dans le vecteur Blue scribe et l'ADN simple brin de chacun de ces plasmides recombinants a été préparé selon la technique décrite par Stragène . La séquence de ces différents fragments clonés a été déterminée selon la technique décrite dans le protocole "séquenase R" version 2 de USB United States Biochemicals. Les fragments séquencés ont été analysés sur gel dénaturant d'acrylamide 6 % (Maniatis et al 1982) le gel est ensuite fixé pendant 10 minutes dans 10 % d'acide acétique puis séché sous vide à 80°C pendant 30 minutes puis mis en autoradiographie (Maniatis et al 1982). La séquence de la protéine de capside de la souche I17F est représentée sur la Fig 3.

La séquence de la protéine de capside est ensuite sortie du plasmide recombinant par digestion aux enzymes de restriction EcoRI et BamHI. Des linkers BamHI sont ajoutés aux extrémités de la séquence (Maniatis et al 1982) qui est ensuite clonée aux sites BamHI du plasmide pBIOS3 déphosphorylé (Maniatis et al 1982). Cette séquence codant pour la protéine de capside est ainsi placée entre le promoteur 35S et le terminateur NOS.

## 3. INTRODUCTION DES GENES CODANT POUR LES PROTEINES DE CAPSIDE DE CMV (SOUCHES I17F ET FNY RESPECTIVEMENT) DANS DES COTYLEDONS DE MELON ET REGENERATION DE PLANTES TRANSGENIQUES

Les 2 gènes codant pur les protéines de capside (I17F et FNY) placés entre des séquences de contrôle sont ensuite clonés séparément dans le vecteur binaire pBI121 (Jefferson et al 1987) modifié par la suppression du site EcoRI 3' du gène GUS, et la création d'un site EcoRI au site HindIII. Ces modifications permettent l'insertion de la cassette promoteur - gène structuré - terminateur issu de pBIOS3.

Ce dernier vecteur est enfin introduit dans la souche LBA 4404. Les souches d'Agrobactérium contenant le vecteur binaire qui possède d'une part la séquence de la protéine de capside de la souche FNY (pBIOS132) et d'autre part de la souche I17F (pBIOS135) sont utilisées pour infecter des cotylédons de melons au stade 2 jours comme décrit dans l'exemple A. Le procédé de régénération des cotylédons transformés est le même que celui décrit dans l'exemple A. Les plantes de melon, régénérées sur le milieu sélectif contenant 100 mg/L kanamycine, sont analysées. Le caractère transgénique des plantes régénérées a été confirmée par la détection de l'activité de G.U.S. et par la technique de formation de cals telle que décrite dans l'exemple A.

L'expression de la protéine de capside de la souche I17F a été détectée chez une plante lors d'une analyse "Western blot" avec des anticorps contre la souche I17F intacte. La protéine de capside exprimée représentait, dans les feuilles, environ 0.01 % de la protéine soluble totale.

Les plantes transformées sont ensuite montées à graines. Ces plantes transformées sont démontrées être résistantes au virus de la mosaïque du concombre.

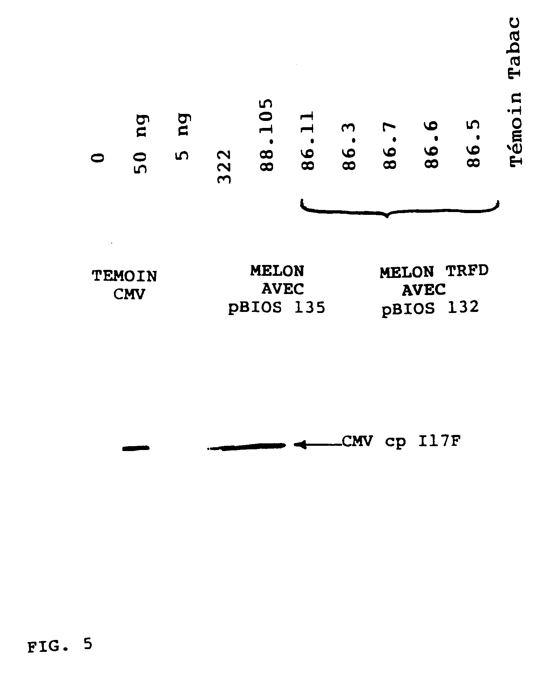

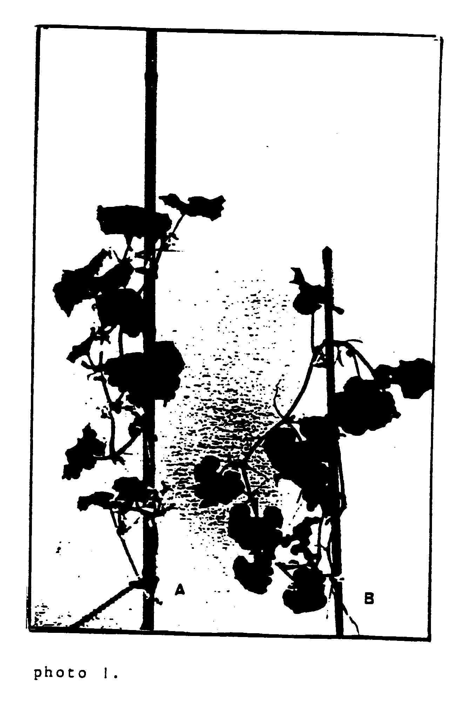

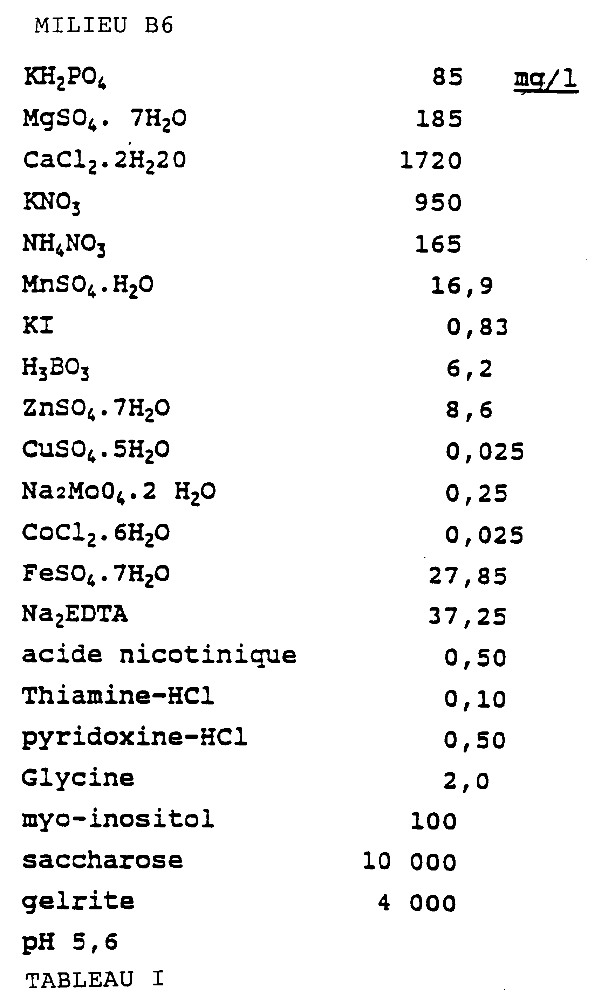

La Figure 5 est un "Western blot" mettant en évidence les bandes immunoréactives détectées avec des anticorps contre le CMV souche I17F.

Quinze plantes R1 ont été testées pour la résistance par inoculation mécanique avec le virus. L'inoculation se fait sur la feuille d'une plantule du stade une feuille. Les symptômes qui apparaissent sur les feuilles formées ensuite permettent d'étudier l'infection systémique du virus. Parmi ces quinze plantes, une dizaine se sont montrées transgéniques par l'expression de GUS.

Chez au moins une de ces plantes transgéniques R1, la quatrième feuille montrait, dix-neuf jours après l'inoculation, une réduction importante des symptômes et une absence de mosaïque, en comparaison avec les témoins Vedrantais non-transformés.

## BIBLIOGRAPHIE

ABAK K, DUMAS DE VAULX R. (1980) - Cucurbit Genetics Cooperative Report 3 : 27-29

ANN G., WATSON B.D., STACHEL S., GORDON M.P., EW N. (1985) - EMBO J. 4 : 277-284.

BEN TAHAR S., DE BOTH M.T.J. (1988) - Introduction of foreign genes into melon (Cucumis melo L.) using Agrobacterium tumefaciens. Proc. Cucurbitaceae. France.

BEVAN M. (1984) - Nucleic Acid Research 12 : 8711-8718.

BOUABDALLAH L., BRANCHARD M. (1986) - Z. Pflanzenzüchtung 96 : 82-85.

BRANCHARD M., CHATEAU M. (1988) - C.R. Acad. Sci Paris 307, Série III : 777-780.

BROADBENT L. (1976) - Ann. Rev. Phytopathol. 14 : 75.

CADE R.M., WEHNER T.C., BLAZICH (1988) - Cucurbit Genetics Cooperative Report 11 : 3-4.

CHEE P.P., TRICOLI D.M. (1988) - Plant Cell Reports 7 : 274-277.

CHILTON M.D., TEPPER D.A., PETIT A., DAVID C., CASSE DELBART F., TEMPE J. (1982) - Nature 295 : 432-434.

COSTA A.S., MULLER G.W. (1980) - Plant Dis. 64 : 538.

CUOZZO M., O'CONNEL K.M., KANIEWSKI W., FANG R.X., CHUA N.H. ET TUMER N.E. (1988) - Biotechnology 6 : 549-557.

DAVID C., CHILTON M.D., TEMPE J. (1984) - Biotechnology 2 : 73-76.

DEAKIN J.R., BOHN G.W., WHITAKER T.W. (1971) - Economic Botany 25 : 195-211.

DE BOTH M.T.J., BEN TAHAR S. (1989) - Proc. Inter. Eucarpia Congress on Genetic Manipulation in Plant Breeding New York : Plenum Press (in press).

DE BOTH M.T.J., (1990) Gene Transfer by electroporation to plant protoplasts and tissues. Thesis University of London.

DEGREEF W., DELON R., DEBLOCK M., LEEMANS J., BOTTERMAN J. (1989) - Biotechnology 7 : 61-64.

DIRKS R., VAN BUGGENUM M. (1989) - Plant Cell Reports 7: 626-627.

FERNOW K.H. (1967) - Phytopathology 57 : 1347.

FRALEY R.T., ROGERS S.G., HORSCH R.B., EICHHOLTZ D.E., FLICK J., FINK C., HOFFMAN N., SANDERS (1985) - Bio Technology 3 : 629-637.

FRALEY R.T., ROGERS S.G., HORSCH R.B. (1986) - Genetic transformation in higher plants. CRC Critical Rev. Plant Sciences 4 : 1-46.

HILDER V.A., GATEHOUSE A.M.R., SHEERMAN S.E., BARKER R.F., BOULTER D. (1987) - Nature 300 : 160-163.

HIDAKA S., TSUNASAWA S., YOON J.O., NARITA K., TAKANAMI Y., KUBO S., MIURA K.I. (1985) - J. Biochem. 97 : 161-171.

HOEKEMA A., HUISMAN M.J., MOLENDIJK L., VAN DE ELZEN P.J.M., CORNELISSEN B.J.C. (1989) - Biotechnology 7 : 273-278.

HORSCH R.B., FRY J.E., HOFFMAN N.L., WALLROTH M., EICHHOLTZ D.Z., ROGERS S.G. (1985) - Science 227 : 1229-1231.

JEFFERSON R.A., KAVANAGH T.A., BEVAN M.W. (1987) - EMBO Journal 6 : 3901-3907.

JELASKA S. (1972) - Planta (Berl.) 103 : 278-280.

JELASKA S. (1974) - Physiol. Plantarum 31 : 257-261.

KATHAL R., BHATNAGAR S.P., BHOJWANI S.T. (1986) - J. Plant Physiol. 126 : 59-62

KATHAL R., BHATNAGAR S.P., BHOJWANI S.T. (1988) - Plant Cell Report 7 : 449-451.

KEVERS C., COUMANS M., COUMANS-GILLES M.F., GASPAR T. (1984) - Physiol. Plant. 61 : 69-74

KHO Y.O., DEN NIJS A.P.M., FRANKEN J. (1980) - Euphytica 29 : 661-672.

KIM S.G., CHANG J.R., CHA H.C., LEE K.W. (1988) - Plant Cell Tissue Organ Culture 12 : 67-74.

KLEE H., HORSCH R., ROGERS S., (1987) - Ann. Rev. Plant Physiol 38 : 467-486.

LASSNER M.W., PETERSON P., YODER, J.I., (1989) Plant. Molec. Rep. 7, 116-128.

LESHEM B., SHALEY D.P., IZHAR S. (1988) - Annals of Botany 61 : 255-260.

MACKAY W.A., NG T.J., HAMMERSCHLAG F.A. (1988) - Cucurbit Genetics Cooperative report 11 : 33-34.

MALEPSY S., NADOLSKA-ORCZYK N. (1983) - Z. Pfanzenphysiologie 111 : 273-276.

MANIATIS T., FRITSCH E.F. ET SAMBROOK J. (1982) - Molecular cloning a laboratory manual.

MARCHOUX G. - Thèse de Docteur ès Science naturelles (1975) - "Propriétés biologiques et génétiques des ARN du virus de la mosaïque du concombre".

MORENO V., GARCIA-SOGO M., GRANELL I., GARCIA-SOGO B., ROIG L.A. (1985) - Plant Cell Tisue and Organ Culture 5: 139-146.

MSIKITA W., SKIRVIN R.M., JUVIK J.A., SPLITTSTOESSER W.E. (1988) - Cucurbit Genetics Cooperative Report 11 : 5-7.

MURASHIGE T., SKOOG F. (1962) - Physiologia Plantarum 15 : 473-497.

NIEDZ R.P., SMITH S.C., DUNBAR K.B., MURAKISHI H.H., STEPHENS C.T. (1989) - Plant Cell Tissue and Organ Culture (in press).

ORIDATE T., OOSAWA K. (1986) - Japan J. Breeding 36 : 424-428.

ORTS M.C., GARCIA-SOGO B., ROCHE M.V., ROIG L.A., MORENO V. (1987) - Hort Science 22 : 666.

POWEL ABEL P., NELSON R.S., DE B., HOFFMANN N., ROGERS S.G., FRALEY R.T., BEACHY R.N. (1986) - Science 232 : 738-743.

RAJASEKARAN K., MULLINS M.G., NAIR Y. (1983) - Annals of Botany 52 : 417-420.

SHARF S.J., HORN G.T., ERLICH H.A. (1986) - Science 233: 1076-1078.

SMITH S., DUNBAR K., NIEDZ R., MURAKISHI H. (1988) - Abstract Proc. Annual TCA Meeting, Las Vegas, Nevada, June 1988.

STABA E.J. (1969) - "Plant tissue culture as a technique for the phytochemist" in "Recent advances in phytochemistry Vol. 2". Ed. : Seikel and Runeckels. Appleton century-crofts, New york.

TUMER N.E., O'CONNEL K.M. NELSON R.S., SANDERS P.R., BEACHY R.N., FRALEY R.T., SHAH D.M.

(1987) - Embo J. 6: 1181-1188.

VAECK M., REYNAERTS A., HOFTE H., JANSENS S., DE BEUCKELEER M., DEAN C., ZABEAU M., VAN MONTAGU M., LEEMANS J. (1987) - Nature 328 : 33-37.

VAN DUN C.M.P., BOL J.F. (1988) - Virology 167 : 649-652.

WEHNER T.C., LOCY R.D. (1981) - HortScience 16 : 759-760.

VAN RAAMSDONK L.W.D. (1989) - Prophyta 6 : 231-232.

**Revendications**

1. Procédé de production de plantules transgéniques ayant un phénotype normal, à partir d'explants génétiquement transformés, lesdites plantules appartenant à l'espèce Cucumis melo et contenant au moins un gène introduit par l'intermédiaire d'Agrobacterium tumefaciens caractérisé par les étapes suivantes :
   i) l'induction de bourgeons génétiquement transformés à partir de cotylédons de Cucumis melo ayant germé pendant 0 à 4 jours et ayant été mis à la suite de ce delai en contact avec A, tumefaciens, l'induction s'effectuant sur un milieu d'induction de bourgeons génétiquement transformés comportant l'ensemble des sels minéraux et vitamines normalement requis pour l'induction de bourgeons à partir d'explants génétiquement non-transformés et contenant, parmi les sels minéraux, environ 440 à environ 2200 mgL$^{-1}$ de chlorure de calcium calculé en CaCl$_2$.2H$_2$O et environ 0,8 à environ 1,2 % de bactoagar ou de agar-agar, ledit milieu d'induction étant additionné d'environ 0,3 à environ 1,13 mgL$^{-1}$ 6-benzyl aminopurine (BAP) et d'environ o a environ 1,3 mgL$^{-1}$ acide indole-3-acetic (AIA) ;
   ii) la culture en deux étapes successives des bourgeons génétiquement transformés ainsi obtenus, la première de ces étapes de culture ayant lieu sur un milieu de culture cellulaire végétale contenant une cytokinine et plus particulièrement de la 6-benzyl aminopurine (BAP), et la deuxième, qui s'effectue lorsque les bourgeons ont atteint au moins environ 3 mm de hauteur, ayant lieu sur un milieu de culture cellulaire végétale contenant comme macro-éléments :

| | | | | | |
|---|---|---|---|---|---|
| KH$_2$PO$_4$ | environ | 50 à | environ | 100 | mgL$^{-1}$ |
| MgSO$_4$ | " | 75 à | " | 300 | " |
| CaCl$_2$.2H$_2$O | " | 500 à | " | 2500 | " |
| KNO$_3$ | " | 750 à | " | 1200 | " |
| NH$_4$NO$_3$ | " | 150 à | " | 200 | " |

2. Procédé selon la revendication 1, caractérisé en ce que le milieu d'induction des bourgeons génétiquement transformés est le milieu de Murashige et Skoog (1962) connu sous le nom de milieu MS, additionné de 0.3 à 1.13 mgL$^{-1}$ BAP, et plus particulièrement de 0.85 mgL$^{-1}$, ladite BAP étant la seule hormone végétale présente.

3. Procédé selon la revendication 1, caractérisé en ce que les vitamines présentes dans le milieu d'induction des bourgeons sont les vitamines de 'Staba' (1969).

4. Procédé selon la revendication 1, caractérisé en ce que la teneur en calcium est d'environ 1000 à environ 2200 mgL$^{-1}$, plus particulièrement d'environ 1750 à environ 2200 mgL$^{-1}$.

5. Procédé selon la revendication 3, caractérisé en ce que le milieu d'induction est le milieu appelé "Milieu M.I" ayant la composition suivante :

### Milieu M.I.

| Macro-éléments : | $KNO_3$ | 1900 | $mgL^{-1}$ |
|---|---|---|---|
| | $NH_4NO_3$ | 1650 | |
| | $CaCl_2.2H_2O$ | 2200 | |
| | $MgSO_4.7H_2O$ | 370 | |
| | $KH_2PO_4$ | 170 | |

| $Na_2EDTA$ | idem MS | |
|---|---|---|
| Micro-éléments | idem MS | |
| Vitamines | Staba | |
| Myo-inositol | 100 | $mgL^{-1}$ |
| Saccharose | 30 | $gL^{-1}$ |

| Agar-agar | 0.8 % | |
|---|---|---|
| BAP | 3.75 | $\mu M$ |
| ABA | 1 | $\mu M$ |

**6.** Procédé selon la revendication 1, caractérisé en ce que le milieu de culture utilisé dans la deuxième étape de culture des bourgeons transformés contient comme macro-éléments:

| $KH_2PO_4$ | 85 mg $L^{-1}$ |
|---|---|
| $MgSO_4$ | 185 mgL$^{-1}$ |
| $CaCl_2.2H_2O$ | 1720 mgL$^{-1}$ |
| $KNO_3$ | 950 mgL$^{-1}$ |
| $NH_4NO_3$ | 165 mgL$^{-1}$ |

**7.** Procédé selon la revendication 2 caractérisé en ce que le milieu est le milieu MB6 ayant la composition indiquée ci-dessous :

15

|  | mg/l |
|---|---|
| KH$_2$PO$_4$ | 85 |
| MgSO$_4$. 7H$_2$O | 185 |
| CaCl$_2$.2H$_2$20 | 1720 |
| KNO$_3$ | 950 |
| NH$_4$NO$_3$ | 165 |
| MnSO$_4$.H$_2$O | 16,9 |
| KI | 0,83 |
| H$_3$BO$_3$ | 6,2 |
| ZnSO$_4$.7H$_2$O | 8,6 |
| CuSO$_4$.5H$_2$O | 0,025 |
| Na$_2$MoO$_4$.2 H$_2$O | 0,25 |
| CoCl$_2$.6H$_2$O | 0,025 |
| FeSO$_4$.7H$_2$O | 27,85 |
| Na$_2$EDTA | 37,25 |
| acide nicotinique | 0,50 |
| Thiamine-HCl | 0,10 |
| pyridoxine-HCl | 0,50 |
| Glycine | 2,0 |
| myo-inositol | 100 |
| saccharose | 10 000 |
| gelrite | 4 000 |
| pH 5,6 | |

8. Procédé selon l'une quelconque des revendications 1 à 7, caractérisé en ce que lesdits cotylédons sont obtenus par la transformation par Agrobacterium tumefaciens de cotylédons issus d'embryons isolés de graines, lesdits cotylédons ayant germé pendant 0 à 3 jours, et plus particulièrement pendant 2 jours.

9. Procédé selon la revendication 8, caractérisé en ce que la transformation des cotylédons s'effectue par mise en contact de fragments de cotylédons avec une suspension bactérienne d'A. tumefaciens, le A. tumefaciens contenant un gène fonctionnel destiné à être introduit dans la plante, suivie de co-culture des fragments de cotylédons et des bactéries pendant environ 48 heures

10. Procédé selon la revendication 9, caractérisé en ce que la suspension bactérienne est une suspension de bactéries dans du milieu MS, et le milieu de co-culture est du milieu MS solidifié avec du agar-agar.

11. Procédé de production de plantes transgéniques appartenant à l'espèce Cucumis melo ayant un phenotype normal, caractérisé en ce que les plantules transgéniques susceptibles d'être produites selon l'une quelconque des revendications 1 a 10 et ayant des racines secondaires sont transférés sur un milieu de culture normalement requis pour le développement de plantes non-génétiquement transformées à partir de plantules.

12. Procédé selon l'une quelconque des revendications précédentes caractérisé en ce que le gène destiné à être introduit dans la plante par l'intermédiaire de Agrobacterium tumefaciens est le gène codant pour

16

la protéine de capside du virus de la mosaïque du concombre (CMV)

13. Procédé selon la revendication 12, caractérisé en ce que le gène comporte la séquence suivante ou un fragment de cette séquence apte à conférer de la résistance au virus de la mosaïque du concombre :

```
GTTATTGTCTACTGACTATATAGAGAGTGTTTGTGCTGTGTTTTCTCTTTT          51

GTGTCGTAGAATTGAGTCGAGTC ATG GAC AAA TCT GAA TCA ACC          95
                        Met Asp Lys Ser Glu Ser Thr          7

AGT GCT GGT CGT AAC CGT CGA CGT CGT CCG CGT CGT GGT          134
Ser Ala Gly Arg Asn Arg Arg Arg Arg Pro Arg Arg Gly          20

TCC CGC TCC GCC CCC TCC TCC GCG GAT GCT AAC TTT AGA          173
Ser Arg Ser Ala Pro Ser Ser Ala Asp Ala Asn Phe Arg          33

GTC TTG TCG CAG CAG CTT TCG CGA CTT AAT AAG ACG TTA          212
Val Leu Ser Gln Gln Leu Ser Arg Leu Asn Lys Thr Leu          46

GCA GCT GGT CGT CCA ACT ATT AAC CAC CCA ACC TTT GTA          251
Ala Ala Gly Arg Pro Thr Ile Asn His Pro Thr Phe Val          59

GGG AGT GAA CGC TGT AGA CCT GGG TAC ACG TTC ACA TCT          290
Gly Ser Glu Arg Cys Arg Pro Gly Tyr Thr Phe Thr Ser          72

ATT ACC CTA AAG CCA CCA AAA ATA GAC CGT GGG TCT TAT          329
Ile Thr Leu Lys Pro Pro Lys Ile Asp Arg Gly Ser Tyr          85

TAC GGT AAA AGG TTG TTA CTA CCT GAT TCA GTC ACG GAA          368
Tyr Gly Lys Arg Leu Leu Leu Pro Asp Ser Val Thr Glu          98

TAT GAT AAG AAG CTT GTT TCG CGC ATT CAA ATT CGA GTT          407
Tyr Asp Lys Lys Leu Val Ser Arg Ile Gln Ile Arg Val          111

AAT CCT TTG CCG AAA TTT GAT TCT ACC GTG TGG GTG ACA          446
Asn Pro Leu Pro Lys Phe Asp Ser Thr Val Trp Val Thr          124

GTC CGT AAA GTT CCT GCC TCC TCG GAC TTA TCC GTT GCC          485
Val Arg Lys Val Pro Ala Ser Ser Asp Leu Ser Val Ala          137

GCC ATC TCT GCT ATG TTC GCG GAC GGA GCC TCA CCG GTA          524
Ala Ile Ser Ala Met Phe Ala Asp Gly Ala Ser Pro Val          150
```

```
CTG GTT TAT CAG TAT GCC GCA TCT GGA GTC CAA GCC AAC    563
Leu Val Tyr Gln Tyr Ala Ala Ser Gly Val Gln Ala Asn    163


AAC AAA CTG TTG TAT GAT CTT TCG GCG ATG CGC GCT GAT    602
Asn Lys Leu Leu Tyr Asp Leu Ser Ala Met Arg Ala Asp    176


ATA GGT GAC ATG AGA AAG TAC GCC GTC CTC GTG TAT TCA    641
Ile Gly Asp Met Arg Lys Tyr Ala Val Leu Val Tyr Ser    189


AAA GAC GAT GCG CTC GAG ACG GAC GAG CTA GTA CTT CAT    680
Lys Asp Asp Ala Leu Glu Thr Asp Glu Leu Val leu His    202

GTT GAC ATC GAG CAC CAA CGC ATT CCC ACA TCT GGA GTG    719
Val Asp Ile Glu His Gln Arg Ile Pro Thr Ser Gly Val    215


CTC CCA GTC TGATTCCGTGTTCCCAGAATCCTCCCTCCGATCTCTGTGG   768
Leu Pro Val                                            218


CGGGAGCTGAGTTGGCAGTTCTGCTATAAACTGTCTGAAGTCACTAAACGTT   820


TTTTACGGTGAACGGGTTGTCCATCCAGCTTACGGCTAAAATGGTCAGTCGT   872


GGAGAAATCCACGCCAGCAGATTTACAAATCTCTGAGGCGCCTTTGAAACCA   924


TCTCCTAGGTTTCTTCGGAAGGACTTCGGTCCGTGTACCTCTAGCACAACGT   976
```

**14.** Procédé selon la revendication 12, caractérisé en ce que le gène comporte la séquence suivante ou un fragment de cette séquence apte à conférer de la résistance au virus de la mosaïque du concombre :

```
AGAGAGTGTGTGTGCTGTGTTTTCTCTTTTGTGTCGTAGAATTGAGTCGAG          51

TC ATG GAC AAA TCT GAA TCA ACC AGT GCT GGT CGT AAC           89
   Met Asp Lys Ser Glu Ser Thr Ser Ala Gly Arg Asn          12

CGT CGA CGT CGT CCG CGT CGT GGT TCC CGC TCC GCC CCC          128
Arg Arg Arg Arg Pro Arg Arg Gly Ser Arg Ser Ala Pro          25

TCC TCC GCG GAT GCT AAC TTT AGA GTC TTG TCG CAG CAG          167
Ser Ser Ala Asp Ala Asn Phe Arg Val Leu Ser Gln Gln          38

CTT TCG CGA CTT AAT AAG ACG TTA GCA GCT GGT CGT CCA          206
Leu Ser Arg Leu Asn Lys Thr Leu Ala Ala Gly Arg Pro          51

ACT ATT AAC CAC CCA ACC TTT GTA GGG AGT GAA CGC TGT          245
Thr Ile Asn His Pro Thr Phe Val Gly Ser Glu Arg Cys          64

AGA CCT GGG TAC ACG TTC ACA TCT ATT ACC CTA AAG CCA          284
Arg Pro Gly Tyr Thr Phe Thr Ser Ile Thr Leu Lys Pro          77

CCA AAA ATA GAC CGT GGG TCT TAT TAC GGT AAA AGG TTG          323
Pro Lys Ile Asp Arg Gly Ser Tyr Tyr Gly Lys Arg Leu          90

TTA CTA CCT GAT TCA GTC ACG GAA TAT GAT AAG AAG CTT          362
Leu Leu Pro Asp Ser Val Thr Glu Tyr Asp Lys Lys Leu          103

GTT TCG CGC ATT CAA ATT CGA GTT AAT CCT TTG CCG AAA          401
Val Ser Arg Ile Gln Ile Arg Val Asn Pro Leu Pro Lys          116

TTT GAT TCT ACC GTG TGG GTG ACA GTC CGT AAA GTT CCT          440
Phe Asp Ser Thr Val Trp Val Thr Val Arg Lys Val Pro          129

GCC TCC TCG GAC TTA TCC GTT GCC GCC ATC TCT GCT ATG          479
Ala Ser Ser Asp Leu Ser Val Ala Ala Ile Ser Ala Met          142

TTC GCG GAC GGA GCC TCA CCG GTA CTG GTT TAT CAG TAT          518
Phe Ala Asp Gly Ala Ser Pro Val Leu Val Tyr Gln Tyr          155

GCC GCA TCT GGA GTC CAA GCC AAC AAC AAA CTG TTG TAT          557
Ala Ala Ser Gly Val Gln Ala Asn Asn Lys Leu Leu Tyr          168

GAT CTT TCG GCG ATG CGC GCT GAT ATA GGT GAC ATG AGA          596
Asp Leu Ser Ala Met Arg Ala Asp Ile Gly Asp Met Arg          181
```

```
AAG TAC GCC GTC CTC GTG TAT TCA AAA GAC GAT GCG CTA    635
Lys Tyr Ala Val Leu Val Tyr Ser Lys Asp Asp Ala Leu    194

GAG ACG GAC GAG CTA GTA CTT CAT GTT GAC ATC GAG CAC    674
Glu Thr Asp Glu Leu Val Leu His Val Asp Ile Glu His    207

CAA CGC ATT CCC ACG TCT GGA GTG CTC CCA GTC TGATTCGT   715
Gln Arg Ile Pro Thr Ser Gly Val Leu Pro Val            218

GTTCCCAGAATCCTCCCTCCGATCTCTGTGGCGGGAGCTGAGTTGGCAGTTC   767

TGCTATAAACTGTCTGAAGTCACTAAACGTTTTTACGGTGAACGGGTTGTCC   819

ATCCAGCTTACGGCTAAAATGGTCAGTCGTGGAGAAATCCACGCCAGTAGAT   871

TTACAAATCTCTGAGGCGCCTTTGAAACCATCTCCTAGGTTTCTTCGGAAGG   923

ACTTCGGTCCGTGTACCTCTAGCACAACGTGCTAGTTTCAGGGTACGGGTGC   975

CCCCCCACTTTCGTGGGGGCCTCCAAAAGGAG                      1007
```

15. Milieu de culture cellulaire apte au développement de bourgeons en plantules au cours de la régénération d'une plante caractérisé en ce qu'il contient comme macro-éléments :

$$
\begin{array}{lllll}
KH_2PO_4 & \text{environ} & 50 \text{ à} & \text{environ} & 100 \text{ mgL}^{-1} \\
MgSO_4.7H_2O & " & 75 \text{ à} & " & 300 \quad " \\
CaCl_2.2H_2O & " & 1000 \text{ à} & " & 2500 \quad " \\
KNO_3 & " & 750 \text{ à} & " & 1200 \quad " \\
NH_4NO_3 & " & 150 \text{ à} & " & 200 \quad "
\end{array}
$$

16. Milieu de culture cellulaire selon la revendication 15, caractérisé en ce qu'il contient comme macro-eléments :

$KH_2PO_4$     85 mgL$^{-1}$
$MgSO_4.7H_2O$     185 mgL$^{-1}$
$CaCl_2.2H_2O$     1720 mgL$^{-1}$
$KNO_3$     950 mgL$^{-1}$
$NH_4NO_3$     165 mgL$^{-1}$

17. Milieu de culture cellulaire selon la revendication 15 caractérisé en ce qu'il a la composition suivante :

| | |
|---|---|
| $KH_2PO_4$ | 85   mg/l |
| $MgSO_4 . 7H_2O$ | 185 |
| $CaCl_2 . 2H_2O$ | 1720 |
| $KNO_3$ | 950 |
| $NH_4NO_3$ | 165 |
| $MnSO_4 . H_2O$ | 16,9 |
| $KI$ | 0,83 |
| $H_3BO_3$ | 6,2 |
| $Z_nSO_4 . 7H_2O$ | 8,6 |
| $C_uSO_4 . 5H_2O$ | 0,025 |
| $Na_2MoO_4 . 2 H_2O$ | 0,25 |
| $CoCl_2 . 6H_2O$ | 0,025 |
| $FeSO_4 . 7H_2O$ | 27,85 |
| $Na_2EDTA$ | 37,25 |
| acide nicotinique | 0,50 |
| Thiamide-HCl | 0,10 |
| pyridoxine-HCl | 0,50 |
| Clycine | 2,0 |
| myo-inositol | 100 |
| saccharose | 10000 |
| gelrite | 4000 |
| pH 5,6 | |

18. Milieu d'induction de bourgeons composé d'un milieu de culture cellulaire végétale comportant l'ensemble des sels minéraux et de vitamines normalement requis pour l'induction de bourgeons à partir d'explants non-génétiquement transformés et contenant parmi ses sels minéraux du chlorure de calcium, et du bacto-agar ou agar-agar, caractérisé en ce que la teneur de ce milieu en $CaCl_2$ est de 1000 à 2200 $mgL^{-1}$ calculé en $CaCl_2$. $H_2O$ et en bacto-agar ou agar-agar est 0,8 à 1,2 %, ledit milieu étant additionné de 0.3 à 2.0 $mgL^{-1}$ BAP et de 0 à 1.3 $mgL^{-1}$ AIA.

19. Milieu d'induction de bourgeons selon la revendication 18, additionné de 0.3 à 1.13 $mgL^{-1}$ BAP, plus particulièrement de 0.85 $mgL^{-1}$, ladite BAP étant la seule hormone végétale présent.

20. Milieu de culture selon la revendication 18 caractérisé en ce que les vitamines présentes dans le milieu d'induction sont les vitamines de 'Staba' (1969).

21. Milieu d'induction de bourgeons selon la revendication 18, caractérisé en ce que la teneur en calcium est d'environ 1750 à environ 2200 $mgL^{-1}$.

22. Milieu d'induction de bourgeons selon la revendication 20, caractérisé en ce qu'il a la composition suivante :

## Milieu M.I.

| Macro-éléments : | KNO$_3$ | 1900 | mgL$^{-1}$ |
| | NH$_4$NO$_3$ | 1650 | |
| | CaCl$_2$.2H$_2$O | 2200 | |
| | MgSO$_4$.7H$_2$O | 370 | |
| | KH$_2$PO$_4$ | 170 | |
| NA$_2$EDTA | idem MS | | |
| Micro-éléments | idem MS | | |
| Vitamines | Staba | | |
| Myo-inositol | 100 | mgL$^{-1}$ | |
| Saccharose | 30 | gL$^{-1}$ | |
| Agar-agar | 0.8 % | | |
| BAP | 3.75 $\mu$M | | |
| ABA | 1 $\mu$M | | |

23. Bourgeons génétiquement transformés appartenant à l'espèce <u>Cucumis melo</u> et susceptibles d'être obtenus au cours du procédé selon l'une quelconque des revendications 8 à 14.

24. Cotylédons génétiquement transformés appartenant à l'espèce <u>Cucumis melo</u> et susceptibles d'être obtenus au cours du procédé selon l'une quelconque des revendications 8 à 14.

25. Plantules transgéniques appartenant à l'espèce <u>Cucumis melo</u>, ayant un phénotype normal, et susceptibles d'être obtenues selon l'une quelconque des revendications 1 à 10.

26. Plantes transgéniques appartenant à l'espèce <u>cucumis melo</u>, ayant un phénotype normal et susceptibles d'être obtenues selon la revendication 11.

27. Melons transgéniques pouvant être produits selon la revendication 11, et ayant un phénotype normal caractérisés en ce qu'ils expriment la protéine de capside du virus de mosaïque de concombre.

28. Semences de plantes transgéniques selon la revendication 26.

## Claims

1. Process for the production of transgenic, phenotypically normal plantlets from genetically transformed explants, said plantlets belonging to the species <u>Cucumis melo</u> and containing at least one gene which has been introduced through <u>Agrobacterium tumefaciens</u>, characterized by the following steps:

   i) induction of genetically transformed shoot buds from cotyledons of <u>Cucumis melo</u> which have germinated for 0 to 4 days and, after this period, have been brought into contact with A. tumefaciens, the induction being carried out on an induction medium for genetically transformed shoot buds which comprises all of the mineral salts and vitamins normally required for the induction of shoot buds from genetically non-transformed explants and containing, amongst the mineral salts, approximately 440 to approximately 2200 mg l$^{-1}$ of calcium chloride calculated as CaCl$_2$.2H$_2$O and approximately 0.8 to approximately 1.2% of bacto-agar or agar-agar, said induction medium being supplemented with approximately 0.3 to approximately 1.13 mg l$^{-1}$ of 6-benzylaminopurine (BAP) and approximately 0 to approximately 1.3 mg l$^{-1}$ of 3-indoleacetic acid (IAA);

   ii) culturing the resulting genetically transformed shoot buds in two successive stages, the first of these culture stages taking place on a plant cell culture medium containing a cytokinin and, more particularly, 6-benzylaminopurine (BAP), and the second stage, which is carried out when the shoot buds have reached a length of at least approximately 3 mm, taking place on a plant cell culture medium containing, as macroelements,

$$
\begin{array}{llll}
KH_2PO_4 & \text{approximately} & 50 \text{ to approximately} & 100 \text{ mg } l^{-1} \\
MgSO_4 & " & 75 \text{ to } " & 300 \quad " \\
CaCl_2 \cdot 2H_2O & " & 500 \text{ to } " & 2500 \quad " \\
KNO_3 & " & 750 \text{ to } " & 1200 \quad " \\
NH_4NO_3 & " & 150 \text{ to } " & 200 \quad " \\
\end{array}
$$

2. Process according to Claim 1, characterized in that the medium for inducing the genetically transformed shoot buds is the medium of Murashige and Skoog (1962), which is known as MS-medium, supplemented with 0.3 to 1.13 mg $l^{-1}$ of BAP and, more particularly, 0.85 mg $l^{-1}$, said BAP being the only plant hormone present.

3. Process according to Claim 1, characterized in that the vitamins present in the shoot bud induction medium are the vitamins of 'Staba' (1969).

4. Process according to Claim 1, characterized in that the calcium content is from approximately 1000 to approximately 2200 mg $l^{-1}$, more particularly from approximately 1750 to approximately 2200 mg $l^{-1}$.

5. Process according to Claim 3, characterized in that the induction medium is the medium termed 'MI-medium', which has the following composition :

**MI-medium**

| | | |
|---|---|---|
| Macroelements: | $KNO_3$ | 1900 mg $l^{-1}$ |
| | $NH_4NO_3$ | 1650 |
| | $CaCl_2.2H_2O$ | 2200 |
| | $MgSO_4.7H_2O$ | 370 |
| | $KH_2PO_4$ | 170 |
| Na$_2$EDTA | as in MS | |
| Microelements | as in MS | |
| Vitamins | Staba | |
| Myo-inositol | 100 mg $l^{-1}$ | |
| Sucrose | 30 g $l^{-1}$ | |
| Agar-agar | 0.8% | |
| BAP | 3.75 $\mu$M | |
| ABA | 1 $\mu$M | |

6. Process according to Claim 1, characterized in that the culture medium used in the second stage of culturing the transformed buds contains, as macro-elements:

KH$_2$PO$_4$      85 mg $l^{-1}$
MgSO$_4$      185 mg $l^{-1}$
CaCl$_2$.2H$_2$O      1720 mg $l^{-1}$
KNO$_3$      950 mg $l^{-1}$
NH$_4$NO$_3$      165 mg $l^{-1}$

7. Process according to Claim 2, characterized in that the medium is the MB6-medium, whose composition is as shown below:

|  | mg/l |
|---|---|
| $KH_2PO_4$ | 85 |
| $MgSO_4 . 7H_2O$ | 185 |
| $CaCl_2 . 2H_2 2O$ | 1720 |
| $KNO_3$ | 950 |
| $NH_4NO_3$ | 165 |
| $MnSO_4 . H_2O$ | 16.9 |
| KI | 0.83 |
| $H_3BO_3$ | 6.2 |
| $ZnSO_4 . 7H_2O$ | 8.6 |
| $CuSO_4 . 5H_2O$ | 0.025 |
| $Na_2MoO_4 . 2H_2O$ | 0.25 |
| $CoCl_2 . 6H_2O$ | 0.025 |
| $FeSO_4 . 7H_2O$ | 27.85 |
| $Na_2EDTA$ | 37.25 |
| Nicotinic acid | 0.50 |
| Thiamine-HCl | 0.10 |
| Pyridoxine-HCl | 0.50 |
| Glycine | 2.0 |
| Myo-inositol | 100 |
| Sucrose | 10,000 |
| Gelrite | 4,000 |

pH 5.6

8. Process according to any one of Claims 1 to 7, characterized in that said cotyledons are obtained by transforming cotyledons from embryos isolated from seeds with Agrobacterium tumefaciens, said cotyledons having germinated for 0 to 3 days, more particularly for 2 days.

9. Process according to Claim 8, characterized in that the transformation of the cotyledons is carried out by bringing cotyledon fragments into contact with a bacterial suspension of A. tumefaciens, where A. tumefaciens contains a functional gene intended to be introduced into the plant, followed by co-culture of the cotyledon fragments and the bacteria over approximately 48 hours.

10. Process according to Claim 9, characterized in that the bacterial suspension is a suspension of bacteria in MS-medium, and the co-culture medium is MS-medium which has been solidified with agar-agar.

11. Method of producing transgenic, phenotypically normal plants which belong to the species Cucumis melo, characterized in that the transgenic plantlets which can be produced by any one of Claims 1 to 10 and which have secondary roots are transferred to a culture medium which is normally required for the development of non-geneti- cally transformed plants from plantlets.

12. Process according to any one of the preceding claims, characterized in that the gene intended to be introduced into the plant through Agrobacterium tumefaciens is the gene which encodes the coat protein of cucumber mosaic virus (CMV).

13. Process according to Claim 12, characterized in that the gene comprises the following sequence or a fragment of this sequence which is capable of imparting resistance to cucumber mosaic virus:

24

```
GTTATTGTCTACTGACTATATAGAGAGTGTTTGTGCTGTGTTTTCTCTTTT          51

GTGTCGTAGAATTGAGTCGAGTC ATG GAC AAA TCT GAA TCA ACC          95
                        Met Asp Lys Ser Glu Ser Thr           7


AGT GCT GGT CGT AAC CGT CGA CGT CGT CCG CGT CGT GGT         134
Ser Ala Gly Arg Asn Arg Arg Arg Arg Pro Arg Arg Gly          20


TCC CGC TCC GCC CCC TCC TCC GCG GAT GCT AAC TTT AGA         173
Ser Arg Ser Ala Pro Ser Ser Ala Asp Ala Asn Phe Arg          33


GTC TTG TCG CAG CAG CTT TCG CGA CTT AAT AAG ACG TTA         212
Val Leu Ser Gln Gln Leu Ser Arg Leu Asn Lys Thr Leu          46


GCA GCT GGT CGT CCA ACT ATT AAC CAC CCA ACC TTT GTA         251
Ala Ala Gly Arg Pro Thr Ile Asn His Pro Thr Phe Val          59


GGG AGT GAA CGC TGT AGA CCT GGG TAC ACG TTC ACA TCT         290
Gly Ser Glu Arg Cys Arg Pro Gly Tyr Thr Phe Thr Ser          72


ATT ACC CTA AAG CCA CCA AAA ATA GAC CGT GGG TCT TAT         329
Ile Thr Leu Lys Pro Pro Lys Ile Asp Arg Gly Ser Tyr          85


TAC GGT AAA AGG TTG TTA CTA CCT GAT TCA GTC ACG GAA         368
Tyr Gly Lys Arg Leu Leu Leu Pro Asp Ser Val Thr Glu          98

TAT GAT AAG AAG CTT GTT TCG CGC ATT CAA ATT CGA GTT         407
Tyr Asp Lys Lys Leu Val Ser Arg Ile Gln Ile Arg Val         111


AAT CCT TTG CCG AAA TTT GAT TCT ACC GTG TGG GTG ACA         446
Asn Pro Leu Pro Lys Phe Asp Ser Thr Val Trp Val Thr         124


GTC CGT AAA GTT CCT GCC TCC TCG GAC TTA TCC GTT GCC         485
Val Arg Lys Val Pro Ala Ser Ser Asp Leu Ser Val Ala         137


GCC ATC TCT GCT ATG TTC GCG GAC GGA GCC TCA CCG GTA         524
Ala Ile Ser Ala Met Phe Ala Asp Gly Ala Ser Pro Val         150
```

```
CTG GTT TAT CAG TAT GCC GCA TCT GGA GTC CAA GCC AAC      563
Leu Val Tyr Gln Tyr Ala Ala Ser Gly Val Gln Ala Asn      163


AAC AAA CTG TTG TAT GAT CTT TCG GCG ATG CGC GCT GAT      602
Asn Lys Leu Leu Tyr Asp Leu Ser Ala Met Arg Ala Asp      176


ATA GGT GAC ATG AGA AAG TAC GCC GTC CTC GTG TAT TCA      641
Ile Gly Asp Met Arg Lys Tyr Ala Val Leu Val Tyr Ser      189


AAA GAC GAT GCG CTC GAG ACG GAC GAG CTA GTA CTT CAT      680
Lys Asp Asp Ala Leu Glu Thr Asp Glu Leu Val leu His      202

GTT GAC ATC GAG CAC CAA CGC ATT CCC ACA TCT GGA GTG      719
Val Asp Ile Glu His Gln Arg Ile Pro Thr Ser Gly Val      215


CTC CCA GTC TGATTCCGTGTTCCCAGAATCCTCCCTCCGATCTCTGTGG     768
Leu Pro Val                                              218


CGGGAGCTGAGTTGGCAGTTCTGCTATAAACTGTCTGAAGTCACTAAACGTT     820


TTTTACGGTGAACGGGTTGTCCATCCAGCTTACGGCTAAAATGGTCAGTCGT     872


GGAGAAATCCACGCCAGCAGATTTACAAATCTCTGAGGCGCCTTTGAAACCA     924


TCTCCTAGGTTTCTTCGGAAGGACTTCGGTCCGTGTACCTCTAGCACAACGT     976
```

**14.** Process according to Claim 12, characterized in that the gene comprises the following sequence or a fragment of this sequence which is capable of imparting resistance to cucumber mosaic virus:

```
AGAGAGTGTGTGTGCTGTGTTTTCTCTTTGTGTCGTAGAATTGAGTCGAG        51


TC ATG GAC AAA TCT GAA TCA ACC AGT GCT GGT CGT AAC         89
   Met Asp Lys Ser Glu Ser Thr Ser Ala Gly Arg Asn         12


CGT CGA CGT CGT CCG CGT CGT GGT TCC CGC TCC GCC CCC        128
Arg Arg Arg Arg Pro Arg Arg Gly Ser Arg Ser Ala Pro        25


TCC TCC GCG GAT GCT AAC TTT AGA GTC TTG TCG CAG CAG        167
Ser Ser Ala Asp Ala Asn Phe Arg Val Leu Ser Gln Gln        38


CTT TCG CGA CTT AAT AAG ACG TTA GCA GCT GGT CGT CCA        206
Leu Ser Arg Leu Asn Lys Thr Leu Ala Ala Gly Arg Pro        51


ACT ATT AAC CAC CCA ACC TTT GTA GGG AGT GAA CGC TGT        245
Thr Ile Asn His Pro Thr Phe Val Gly Ser Glu Arg Cys        64


AGA CCT GGG TAC ACG TTC ACA TCT ATT ACC CTA AAG CCA        284
Arg Pro Gly Tyr Thr Phe Thr Ser Ile Thr Leu Lys Pro        77


CCA AAA ATA GAT CGT GGG TCT TAT TAC GGT AAA AGG TTG        323
Pro Lys Ile Asp Arg Gly Ser Tyr Tyr Gly Lys Arg Leu        90


TTA CTA CCT GAT TCA GTC ACG GAA TAT GAT AAG AAG CTT        362
Leu Leu Pro Asp Ser Val Thr Glu Tyr Asp Lys Lys Leu        103


GTT TCG CGC ATT CAA ATT CGA GTT AAT CCT TTG CCG AAA        401
Val Ser Arg Ile Gln Ile Arg Val Asn Pro Leu Pro Lys        116


TTT GAT TCT ACC GTG TGG GTG ACA GTC CGT AAA GTT CCT        440
Phe Asp Ser Thr Val Trp Val Thr Val Arg Lys Val Pro        129


GCC TCC TCG GAC TTA TCC GTT GCC GCC ATC TCT GCT ATG        479
Ala Ser Ser Asp Leu Ser Val Ala Ala Ile Ser Ala Met        142


TTC GCG GAC GGA GCC TCA CCG GTA CTG GTT TAT CAG TAT        518
Phe Ala Asp Gly Ala Ser Pro Val Leu Val Tyr Gln Tyr        155


GCC GCA TCT GGA GTC CAA GCC AAC AAC AAA CTG TTG TAT        557
Ala Ala Ser Gly Val Gln Ala Asn Asn Lys Leu Leu Tyr        168


GAT CTT TCG GCG ATG CGC GCT GAT ATA GGT GAC ATG AGA        596
Asp Leu Ser Ala Met Arg Ala Asp Ile Gly Asp Met Arg        181
```

EP 0 412 912 B1

```
AAG TAC GCC GTC CTC GTG TAT TCA AAA GAC GAT GCG CTA    635
Lys Tyr Ala Val Leu Val Tyr Ser Lys Asp Asp Ala Leu   194

GAG ACG GAC GAG CTA GTA CTT CAT GTT GAC ATC GAG CAC    674
Glu Thr Asp Glu Leu Val Leu His Val Asp Ile Glu His    207

CAA CGC ATT CCC ACG TCT GGA GTG CTC CCA GTC TGATTCGT   715
Gln Arg Ile Pro Thr Ser Gly Val Leu Pro Val            218

GTTCCCAGAATCCTCCCTCCGATCTCTGTGGCGGGAGCTGAGTTGGCAGTTC   767

TGCTATAAACTGTCTGAAGTCACTAAACGTTTTTACGGTGAACGGGTTGTCC   819

ATCCAGCTTACGGCTAAAATGGTCAGTCGTGGAGAAATCCACGCCAGTAGAT   871

TTACAAATCTCTGAGGCGCCTTTGAAACCATCTCCTAGGTTTCTTCGGAAGG   923

ACTTCGGTCCGTGTACCTCTAGCACAACGTGCTAGTTTCAGGGTACGGGTGC   975

CCCCCCACTTTCGTGGGGGCCTCCAAAAGGAG                       1007
```

15. Cell culture medium suitable for the development of shoot buds into plantlets in the course of the regeneration of a plant, characterized in that it contains, as macro-elements:

```
KH₂PO₄     approximately   50 to approximately   100 mg l⁻¹
MgSO₄.7H₂O      "           75 to        "         300   "
CaCl₂.2H₂O      "         1000 to        "        2500   "
KNO₃            "          750 to        "        1200   "
NH₄NO₃          "          150 to        "         200   "
```

16. Cell culture medium according to Claim 15, characterized in that it contains, as macroelements,

KH₂PO₄        85 mg l⁻¹
MgSO₄.7H₂O    185 mg l⁻¹
CaCl₂.2H₂O    1720 mg l⁻¹
KNO₃          950 mg l⁻¹
NH₄NO₃        165 mg l⁻¹

17. Cell culture medium according to Claim 15, characterized in that it has the following composition:

28

|  | mg/1 |
|---|---|
| $KH_2PO_4$ | 85 |
| $MgSO_4.7H_2O$ | 185 |
| $CaCl_2.2H_2O$ | 1720 |
| $KNO_3$ | 950 |
| $NH_4NO_3$ | 165 |
| $MnSO_4.H_2O$ | 16.9 |
| KI | 0.83 |
| $H_3BO_3$ | 6.2 |
| $ZnSO_4.7H_2O$ | 8.6 |
| $CuSO_4.5H_2O$ | 0.025 |
| $Na_2MoO_4.2H_2O$ | 0.25 |
| $CoCl_2.6H_2O$ | 0.025 |
| $FeSO_4.7H_2O$ | 27.85 |
| $Na_2EDTA$ | 37.25 |
| Nicotinic acid | 0.50 |
| Thiamine-HCl | 0.10 |
| Pyridoxine-HCl | 0.50 |
| Glycine | 2.0 |
| Myo-inositol | 100 |
| Sucrose | 10,000 |
| Gelrite | 4,000 |
| pH 5.6 | |

18. Shoot bud induction medium composed of a plant cell culture medium which comprises all the mineral salts and the vitamins normally required for inducing shoot buds from non-genetically transformed explants and containing, amongst its mineral salts, calcium chloride, and bacto- agar or agar-agar, characterized in that the $CaCl_2$ content of this medium is 1000 to 2200 mg $l^{-1}$ calculated as $CaCl_2.H_2O$, and the bacto-agar or agar-agar content is 0.8 to 1.2%, said medium being supplemented with 0.3 to 2.0 mg $l^{-1}$ of BAP and 0 to 1.3 mg $l^{-1}$ of IAA.

19. Shoot bud induction medium according to Claims 18, supplemented with 0.3 to 1.13 mg $l^{-1}$ of BAP, more particularly 0.85 mg $l^{-1}$, said BAP being the only plant hormone present.

20. Culture medium according to Claim 18, charac- terized in that the vitamins present in the induction medium are the vitamins of 'Staba' (1969).

21. Bud induction medium according to Claim 18, characterized in that the calcium content is from approximately 1750 to approximately 2200 mg $l^{-1}$.

22. Bud induction medium according to Claim 20, characterized in that it has the following composition :

**MI-medium**

Macroelements: 

| | | |
|---|---|---|
| KNO$_3$ | 1900 mg l$^{-1}$ | |
| NH$_4$NO$_3$ | 1650 | |
| CaCl$_2$.2H$_2$O | 2200 | |
| MgSO$_4$.7H$_2$O | 370 | |
| KH$_2$PO$_4$ | 170 | |

Na$_2$EDTA          as in MS

Microelements   as in MS

Vitamins          Staba

Myo-inositol    100 mg l$^{-1}$

Sucrose            30 g l$^{-1}$

Agar-agar        0.8%

BAP                3.75 $\mu$M

ABA                1 $\mu$M

23. Genetically transformed shoot buds which belong to the species <u>Cucumis melo</u> and which can be obtained in the course of the process according to any one of Claims 8 to 14.

24. Genetically transformed cotyledons which belong to the species <u>Cucumis melo</u> and which can be obtained in the course of the process according to any one of Claims 8 to 14.

25. Transgenic, phenotypically normal plantlets which belong to the species <u>Cucumis melo</u> and which can be obtained according to any one of Claims 1 to 10.

26. Transgenic, phenotypically normal plants which belong to the species <u>Cucumis melo</u> and which can be obtained according to Claim 11.

27. Transgenic, phenotypically normal melons which can be produced according to Claim 11, characterized in that they express the coat protein of cucumber mosaic virus.

28. Seed of transgenic plants according to Claim 26.

**Patentansprüche**

1. Verfahren zur Herstellung von einen normalen Phenotyp aufweisenden transgenen Keimlingen von genetisch transformierten Explantaten, wobei die Keimlinge zur Art Cucumis melo gehören und mindestens ein mit Hilfe von Agrobacterium tumefaciens eingeführtes Gen enthalten, gekennzeichnet durch die folgenden Schritte:

i) Induktion von genetisch transformierten Knospen von Cotyledonen von Cucumis melo, die 0 bis 4 Tage gekeimt haben und nach diesem Zeitraum mit A. tumefaciens in Kontakt gebracht wurden, wobei die Induktion in einem Induktionsmedium für genetisch transformierte Knospen erfolgt, welches alle die Mineralsalzen und Vitaminen enthält, die normalerweise zur Induktion von von genetisch nicht transformierten Explantaten stammenden Knospen erforderlich ist und welches unter den Mineralsalzen etwa 440 bis etwa 2200. mg/l Calciumchlorid, berechnet als CaCl$_2$·2H$_2$O, und etwa 0,8 bis etwa 1,2 % Bacto-Agar oder Agar-Agar enthält, wobei zu dem Induktionsmedium etwa 0,3 bis etwa 1,13 mg/l 6-Benzylaminopurin (BAP) und etwa 0 bis etwa 1,3 mg/l Indol-3-essigsäure (AIA) hinzugefügt wurde;

ii) Züchtung der so erhaltenen genetisch transformierten Knospen in zwei aufeinanderfolgenden Schritten, wobei der erste dieser Züchtungsschritte in einem Kulturmedium für Pflanzenzellen stattfindet, das ein Cytokinin und insbesondere 6-Benzylaminopurin (BAP) enthält, und der zweite Züchtungsschritt, der dann stattfindet, wenn die Knospen mindestens etwa 3 mm Höhe erreicht haben, in einem Kulturmedium für Pflanzenzellen stattfindet, das als Macroelemente enthält:

| | |
|---|---|
| $KH_2PO_4$ | etwa 50 bis etwa 100 mg/l |
| $MgSO_4$ | etwa 75 bis etwa 300 mg/l |
| $CaCl_2 \cdot 2H_2O$ | etwa 500 bis etwa 2500 mg/l |
| $KNO_3$ | etwa 750 bis etwa 1200 mg/l |
| $NH_4NO_3$ | etwa 150 bis etwa 200 mg/l. |

2. Verfahren nach Anspruch 1, dadurch gekennzeichnet, daß das Induktionsmedium der genetisch transformierten Knospen Murashige und Skoog (1962)-Medium ist, das unter dem Namen MS-Medium bekannt ist, unter Zusatz von 0,3 bis 1,13 mg/l BAP, insbesondere 0,85 mg/l, wobei das BAP das einzige vorhandene Pflanzenhormon ist

3. Verfahren nach Anspruch 1, dadurch gekennzeichnet, daß die in dem Induktionsmedium von Knospen vorhandenen Vitamine die Vitamine von "Staba" (1969) sind.

4. Verfahren nach Anspruch 1, dadurch gekennzeichnet, daß der Gehalt an Calcium etwa 1000 bis etwa 2200 mg/l, insbesondere etwa 1750 bis etwa 2200 mg/l, beträgt.

5. Verfahren nach Anspruch 3, dadurch gekennzeichnet, daß das Induktionsmedium das "Medium M.I" genannte Medium ist, mit der folgenden Zusammensetzung:

## M.I.-Medium:

| Hauptbestandteile: | | |
|---|---|---|
| | $KNO_3$ | 1900 mg/l |
| | $NH_4NO_3$ | 1650 |
| | $CaCl_2 \cdot 2H_2O$ | 2200 |
| | $MgSO_4 \cdot 7H_2O$ | 370 |
| | $KH_2PO_4$ | 170 |
| $Na_2EDTA$ | idem MS | |
| Spurenelemente | idem MS | |
| Vitamine | Staba | |
| Myo-Inositol | 100 mg/l | |
| Saccharose | 30 g/l | |
| Agar-Agar | 0,8 % | |
| BAP | 3,75 $\mu M$ | |
| ABA | 1 $\mu M$ | |

6. Verfahren nach Anspruch 1, dadurch gekennzeichnet, daß das im zweiten Züchtungsschritt der transformierten Knospen eingesetzte Kulturmedium folgende Macroelemente enthält:

| | |
|---|---|
| $KH_2PO_4$ | 85 mg/l |
| $MgSO_4$ | 185 mg/l |
| $CaCl_2 \cdot 2H_2O$ | 1720 mg/l |
| $KNO_3$ | 950 mg/l |
| $NH_4NO_3$ | 165 mg/l |

7. Verfahren nach Anspruch 2, dadurch gekennzeichnet, daß das Medium das Medium MB6 ist, das nachstehende Zusammensetzung aufweist:

| | | |
|---|---|---|
| $KH_2PO_4$ | 85 | mg/l |
| $MgSO_4 \cdot 7H_2O$ | 185 | mg/l |
| $CaCl_2 \cdot 2H_2O$ | 1720 | mg/l |
| $KNO_3$ | 950 | mg/l |
| $NH_4NO_3$ | 165 | mg/l |
| $MnSO_4 \cdot H_2O$ | 16,9 | mg/l |
| KI | 0,83 | mg/l |
| $H_3BO_3$ | 6,2 | mg/l |
| $ZnSO_4 \cdot 7H_2O$ | 8,6 | mg/l |
| $CuSO_4 \cdot 5H_2O$ | 0,025 | mg/l |
| $Na_2MoO_4 \cdot 2H_2O$ | 0,25 | mg/l |
| $CoCl_2 \cdot 6H_2O$ | 0,025 | mg/l |
| $FeSO_4 \cdot 7H_2O$ | 27,85 | mg/l |
| $Na_2EDTA$ | 37,25 | mg/l |
| Nicotinsäure | 0,50 | mg/l |
| Thiamin-HCl | 0,10 | mg/l |
| Pyridoxin-HCl | 0,50 | mg/l |
| Glycin | 2,0 | mg/l |
| Myo-Inositol | 100 | mg/l |
| Saccharose | 10 000 | mg/l |
| Gelrit | 4 000 | mg/l |
| pH 5,6 | | |

8. Verfahren nach einem der Ansprüche 1 bis 7, dadurch gekennzeichnet, daß die Cotyledonen durch Transformation von Cotyledonen mit Agrobacterium tumefaciens erhalten werden, die von aus Samen isolierten Embryonen stammen, wobei die Cotyledonen 0 bis 3 Tage, insbesondere 2 Tage, gekeimt haben.

9. Verfahren nach Anspruch 8, dadurch gekennzeichnet, daß die Transformation der Cotyledonen durch Inkontaktbringen von Cotyledonfragmenten mit einer bakteriellen Suspension von A. tumefaciens erfolgt, wobei A. tumefaciens ein funktionelles Gen enthält, das zum Einbau in die Pflanze bestimmt ist, gefolgt von einer gleichzeitigen Züchtung der Cotyledonfragmente und der Bakterien für etwa 48 Stunden.

10. Verfahren nach Anspruch 9, dadurch gekennzeichnet, daß die bakterielle Suspension eine Bakteriensuspension in dem Medium MS ist, und das Medium der gleichzeitigen Züchtung MS-Medium ist; das mit Agar-Agar verfestigt wurde.

11. Verfahren zur Herstellung von transgenen Pflanzen der Art Cucumis melo mit einem normalen Phenotyp, dadurch gekennzeichnet, daß die transgenen Keimlinge, die nach einem der Ansprüche 1 bis 10 hergestellt werden können und sekundäre Wurzeln aufweisen, in ein Kulturmedium transferiert werden, das normalerweise zur Entwicklung von nicht genetisch transformierten Pflanzen aus Keim- lingen erforderlich ist.

12. Verfahren nach einem der vorangehenden Ansprüche, dadurch gekennzeichnet, daß das mit Hilfe von Agrobacterium tumefaciens in die Pflanze einzuführende Gen das Gen ist, welches das Capsidprotein des Gurkenmosaikvirus (CMV) codiert.

**13.** Verfahren nach Anspruch 12, dadurch gekennzeichnet, daß das Gen die folgende Sequenz oder ein Fragment dieser Sequenz enthält, das Resistenz gegenüber dem Gurkenmosaikvirus verleihen kann:

```
GTTATTGTCTACTGACTATATAGAGAGTGTTTGTGCTGTGTTTTCTCTTTT          51


GTGTCGTAGAATTGAGTCGAGTC ATG GAC AAA TCT GAA TCA ACC          95
                        Met Asp Lys Ser Glu Ser Thr          7


AGT GCT GGT CGT AAC CGT CGA CGT CGT CCG CGT CGT GGT          134
Ser Ala Gly Arg Asn Arg Arg Arg Arg Pro Arg Arg Gly          20


TCC CGC TCC GCC CCC TCC TCC GCG GAT GCT AAC TTT AGA          173
Ser Arg Ser Ala Pro Ser Ser Ala Asp Ala Asn Phe Arg          33


GTC TTG TCG CAG CAG CTT TCG CGA CTT AAT AAG ACG TTA          212
Val Leu Ser Gln Gln Leu Ser Arg Leu Asn Lys Thr Leu          46


GCA GCT GGT CGT CCA ACT ATT AAC CAC CCA ACC TTT GTA          251
Ala Ala Gly Arg Pro Thr Ile Asn His Pro Thr Phe Val          59


GGG AGT GAA CGC TGT AGA CCT GGG TAC ACG TTC ACA TCT          290
Gly Ser Glu Arg Cys Arg Pro Gly Tyr Thr Phe Thr Ser          72


ATT ACC CTA AAG CCA CCA AAA ATA GAC CGT GGG TCT TAT          329
Ile Thr Leu Lys Pro Pro Lys Ile Asp Arg Gly Ser Tyr          85


TAC GGT AAA AGG TTG TTA CTA CCT GAT TCA GTC ACG GAA          368
Tyr Gly Lys Arg Leu Leu Leu Pro Asp Ser Val Thr Glu          98


TAT GAT AAG AAG CTT GTT TCG CGC ATT CAA ATT CGA GTT          407
Tyr Asp Lys Lys Leu Val Ser Arg Ile Gln Ile Arg Val          111


AAT CCT TTG CCG AAA TTT GAT TCT ACC GTG TGG GTG ACA          446
Asn Pro Leu Pro Lys Phe Asp Ser Thr Val Trp Val Thr          124


GTC CGT AAA GTT CCT GCC TCC TCG GAC TTA TCC GTT GCC          485
Val Arg Lys Val Pro Ala Ser Ser Asp Leu Ser Val Ala          137
```

```
GCC ATC TCT GCT ATG TTC GCG GAC GGA GCC TCA CCG GTA    524
Ala Ile Ser Ala Met Phe Ala Asp Gly Ala Ser Pro Val    150

CTG GTT TAT CAG TAT GCC GCA TCT GGA GTC CAA GCC AAC    563
Leu Val Tyr Gln Tyr Ala Ala Ser Gly Val Gln Ala Asn    163

AAC AAA CTG TTG TAT GAT CTT TCG GCG ATG CGC GCT GAT    602
Asn Lys Leu Leu Tyr Asp Leu Ser Ala Met Arg Ala Asp    176

ATA GGT GAC ATG AGA AAG TAC GCC GTC CTC GTG TAT TCA    641
Ile Gly Asp Met Arg Lys Tyr Ala Val Leu Val Tyr Ser    189

AAA GAC GAT GCG CTC GAG ACG GAC GAG CTA GTA CTT CAT    680
Lys Asp Asp Ala Leu Glu Thr Asp Glu Leu Val leu His    202

GTT GAC ATC GAG CAC CAA CGC ATT CCC ACA TCT GGA GTG    719
Val Asp Ile Glu His Gln Arg Ile Pro Thr Ser Gly Val    215

CTC CCA GTC TGATTCCGTGTTCCCAGAATCCTCCCTCCGATCTCTGTGG   768
Leu Pro Val                                             218

CGGGAGCTGAGTTGGCAGTTCTGCTATAAACTGTCTGAAGTCACTAAACGTT   820

TTTTACGGTGAACGGGTTGTCCATCCAGCTTACGGCTAAAATGGTCAGTCGT   872

GGAGAAATCCACGCCAGCAGATTTACAAATCTCTGAGGCGCCTTTGAAACCA   924

TCTCCTAGGTTTCTTCGGAAGGACTTCGGTCCGTGTACCTCTAGCACAACGT   976
```

**14.** Verfahren nach Anspruch 12, dadurch gekennzeichnet, daß das Gen die folgende Sequenz oder ein Fragment dieser Sequenz enthält, das Resistenz gegenüber dem Gurkenmosaikvirus verleihen kann:

```
AGAGAGTGTGTGTGTGCTGTGTTTTCTCTTTGTGTCGTAGAATTGAGTCGAG          51

TC ATG GAC AAA TCT GAA TCA ACC AGT GCT GGT CGT AAC          89
   Met Asp Lys Ser Glu Ser Thr Ser Ala Gly Arg Asn          12

CGT CGA CGT CGT CCG CGT CGT GGT TCC CGC TCC GCC CCC         128
Arg Arg Arg Arg Pro Arg Arg Gly Ser Arg Ser Ala Pro          25

TCC TCC GCG GAT GCT AAC TTT AGA GTC TTG TCG CAG CAG         167
Ser Ser Ala Asp Ala Asn Phe Arg Val Leu Ser Gln Gln          38

CTT TCG CGA CTT AAT AAG ACG TTA GCA GCT GGT CGT CCA         206
Leu Ser Arg Leu Asn Lys Thr Leu Ala Ala Gly Arg Pro          51

ACT ATT AAC CAC CCA ACC TTT GTA GGG AGT GAA CGC TGT         245
Thr Ile Asn His Pro Thr Phe Val Gly Ser Glu Arg Cys          64

AGA CCT GGG TAC ACG TTC ACA TCT ATT ACC CTA AAG CCA         284
Arg Pro Gly Tyr Thr Phe Thr Ser Ile Thr Leu Lys Pro          77

CCA AAA ATA GAC CGT GGG TCT TAT TAC GGT AAA AGG TTG         323
Pro Lys Ile Asp Arg Gly Ser Tyr Tyr Gly Lys Arg Leu          90

TTA CTA CCT GAT TCA GTC ACG GAA TAT GAT AAG AAG CTT         362
Leu Leu Pro Asp Ser Val Thr Glu Tyr Asp Lys Lys Leu         103

GTT TCG CGC ATT CAA ATT CGA GTT AAT CCT TTG CCG AAA         401
Val Ser Arg Ile Gln Ile Arg Val Asn Pro Leu Pro Lys         116

TTT GAT TCT ACC GTG TGG GTG ACA GTC CGT AAA GTT CCT         440
Phe Asp Ser Thr Val Trp Val Thr Val Arg Lys Val Pro         129

GCC TCC TCG GAC TTA TCC GTT GCC GCC ATC TCT GCT ATG         479
Ala Ser Ser Asp Leu Ser Val Ala Ala Ile Ser Ala Met         142

TTC GCG GAC GGA GCC TCA CCG GTA CTG GTT TAT CAG TAT         518
Phe Ala Asp Gly Ala Ser Pro Val Leu Val Tyr Gln Tyr         155

GCC GCA TCT GGA GTC CAA GCC AAC AAC AAA CTG TTG TAT         557
Ala Ala Ser Gly Val Gln Ala Asn Asn Lys Leu Leu Tyr         168

GAT CTT TCG GCG ATG CGC GCT GAT ATA GGT GAC ATG AGA         596
Asp Leu Ser Ala Met Arg Ala Asp Ile Gly Asp Met Arg         181

AAG TAC GCC GTC CTC GTG TAT TCA AAA GAC GAT GCG CTA         635
Lys Tyr Ala Val Leu Val Tyr Ser Lys Asp Asp Ala Leu         194
```

```
GAG ACG GAC GAG CTA GTA CTT CAT GTT GAC ATC GAG CAC    674
Glu Thr Asp Glu Leu Val Leu His Val Asp Ile Glu His    207


CAA CGC ATT CCC ACG TCT GGA GTG CTC CCA GTC TGATTCGT   715
Gln Arg Ile Pro Thr Ser Gly Val Leu Pro Val            218
```

GTTCCCAGAATCCTCCCTCCGATCTCTGTGGCGGGAGCTGAGTTGGCAGTTC    767

TGCTATAAACTGTCTGAAGTCACTAAACGTTTTTACGGTGAACGGGTTGTCC    819

ATCCAGCTTACGGCTAAAATGGTCAGTCGTGGAGAAATCCACGCCAGTAGAT    871

TTACAAATCTCTGAGGCGCCTTTGAAACCATCTCCTAGGTTTCTTCGGAAGG    923

ACTTCGGTCCGTGTACCTCTAGCACAACGTGCTAGTTTCAGGGTACGGGTGC    975

CCCCCCACTTTCGTGGGGGCCTCCAAAAGGAG                       1007

15. Zellkulturmedium, das zur Entwicklung von Knospen zu Keimlingen im Verlauf der Regeneration einer Pflanze geeignet ist, dadurch gekennzeichnet, daß es folgende Macroelemente enthält:

$$
\begin{array}{lllllll}
KH_2PO_4 & \text{etwa} & 50 & \text{bis} & \text{etwa} & 100 & mg/l \\
MgSO_4 \cdot 7H_2O & \text{etwa} & 75 & \text{bis} & \text{etwa} & 300 & mg/l \\
CaCl_2 \cdot 2H_2O & \text{etwa} & 1000 & \text{bis} & \text{etwa} & 2500 & mg/l \\
KNO_3 & \text{etwa} & 750 & \text{bis} & \text{etwa} & 1200 & mg/l \\
NH_4NO_3 & \text{etwa} & 150 & \text{bis} & \text{etwa} & 200 & mg/l .
\end{array}
$$

16. Zellkulturmedium nach Anspruch 15, dadurch gekennzeichnet, daß es folgende Macroelemente enthält:

| | |
|---|---|
| $KH_2PO_4$ | 85 mg/l |
| $MgSO_4.7H_2O$ | 185 mg/l |
| $CaCl_2 \cdot 2H_2O$ | 1720 mg/l |
| $KNO_3$ | 950 mg/l |
| $NH_4NO_3$ | 165 mg/l |

17. Zellkulturmedium nach Anspruch 15, dadurch gekennzeichnet, daß es folgende Zusammensetzung hat:

36

| | | |
|---|---|---|
| $KH_2PO_4$ | 85 | mg/l |
| $MgSO_4 \cdot 7H_2O$ | 185 | mg/l |
| $CaCl_2 \cdot 2H_2O$ | 1720 | mg/l |
| $KNO_3$ | 950 | mg/l |
| $NH_4NO_3$ | 165 | mg/l |
| $MnSO_4 \cdot H_2O$ | 16,9 | mg/l |
| KI | 0,83 | mg/l |
| $H_3BO_3$ | 6,2 | mg/l |
| $ZnSO_4 \cdot 7H_2O$ | 8,6 | mg/l |
| $CuSO_4 \cdot 5H_2O$ | 0,025 | mg/l |
| $Na_2MoO_4 \cdot 2H_2O$ | 0,25 | mg/l |
| $CoCl_2 \cdot 6H_2O$ | 0,025 | mg/l |
| $FeSO_4 \cdot 7H_2O$ | 27,85 | mg/l |
| $Na_2EDTA$ | 37,25 | mg/l |
| Nicotinsäure | 0,50 | mg/l |
| Thiamin-HCl | 0,10 | mg/l |
| Pyridoxin-HCl | 0,50 | mg/l |
| Glycin | 2,0 | mg/l |
| Myo-Inositol | 100 | mg/l |
| Saccharose | 10 000 | mg/l |
| Gelrit | 4 000 | mg/l |
| pH 5,6 | | |

18. Induktionsmedium für Knospen, zusammengesetzt aus einem Pflanzenzellkulturmedium, das alle die aus Mineralsalzen und Vitaminen enthält, die normalerweise zur Induktion von Knospen von nicht genetisch transformierten Explantaten erforderlich ist, und unter diesen Mineralsalzen Calciumchlorid und Bacto-Agar oder Agar-Agar enthält, dadurch gekennzeichnet, daß der Gehalt des Mediums an Calciumchlorid 1000 bis 2200 mg/l ist, berechnet als $CaCl_2 \cdot 2H_2O$, und an Bacto-Agar oder Agar-Agar 0,8 bis 1,2 % ist, wobei dem Medium 0,3 bis 2,0 mg/l BAP und 0 bis 1,3 mg/l AIA zugefügt wurden.

19. Induktionsmedium für Knospen nach Anspruch 18, mit dem Zusatz von 0,3 bis 1,13 mg/l BAP, insbesondere 0,85 mg/l, wobei das BAP das einzige vorhandene Pflanzenhor- mon ist.

20. Kulturmedium nach Anspruch 18, dadurch gekennzeichnet, daß die in dem Induktionsmedium vorhandenen Vitamine die Vitamine von "Staba" (1969) sind.

21. Induktionsmedium für Knospen nach Anspruch 18, dadurch gekennzeichnet, daß der Gehalt an Calcium etwa 1750 bis etwa 2200 mg/l beträgt.

22. Induktionsmedium für Knospen nach Anspruch 20, dadurch gekennzeichnet, daß es die folgende Zusammensetzung hat:

**M.I.-Medium:**

| | | | |
|---|---|---|---|
| Macroelemente : | $KNO_3$ | 1900 | mg/l |
| | $NH_4NO_3$ | 1650 | |
| | $CaCl_2 \cdot 2H_2O$ | 2200 | |
| | $MgSO_4 \cdot 7H_2O$ | 370 | |
| | $KH_2PO_4$ | 170 | |
| $Na_2EDTA$ | idem MS | | |
| Spurenelemente | idem MS | | |
| Vitamine | Staba | | |
| Myo-Inositol | 100 | mg/l | |
| Saccharose | 30 | g/l | |
| Agar-Agar | 0,8 | % | |
| BAP | 3,75 | $\mu M$ | |
| ABA | 1 | $\mu M$ | |

23. Genetisch transformierte Knopsen, die zur Art Cucumis melo gehören und gemäß dem Verfahren nach einem der Ansprüche 8 bis 14 erhalten werden können.

24. Genetisch transformierte Cotyledonen, die zur Art Cucumis melo gehören und gemäß dem Verfahren nach einem der Ansprüche 8 bis 14 erhalten werden können.

25. Transgene Keimlinge der Art Cucumis melo mit einem normalen Phenotyp, die gemäß einem der Ansprüche 1 bis 10 erhalten werden können.

26. Transgene Pflanzen der Art Cucumis melo mit einem normalen Phenotyp, die nach Anspruch 11 erhalten werden können.

27. Transgene Melonen, die nach Anspruch 11 erhalten werden können, mit einem normalen Phenotyp, dadurch gekennzeichnet, daß sie das Capsidprotein des Gurkenmosaikvirus exprimieren.

28. Samen von transgenen Pflanzen nach Anspruch 26.

FIG. 1  : Influence de l'âge des cotylédons
          sur le pourcentage de régénération
          chez des génotypes différents.

```
GTTATTGTCTACTGACTATATAGAGAGTGTTTGTGCTGTGTTTTCTCTTTT        51


GTGTCGTAGAATTGAGTCGAGTC ATG GAC AAA TCT GAA TCA ACC        95
                        Met Asp Lys Ser Glu Ser Thr         7


AGT GCT GGT CGT AAC CGT CGA CGT CGT CCG CGT CGT GGT       134
Ser Ala Gly Arg Asn Arg Arg Arg Arg Pro Arg Arg Gly        20


TCC CGC TCC GCC CCC TCC TCC GCG GAT GCT AAC TTT AGA       173
Ser Arg Ser Ala Pro Ser Ser Ala Asp Ala Asn Phe Arg        33


GTC TTG TCG CAG CAG CTT TCG CGA CTT AAT AAG ACG TTA       212
Val Leu Ser Gln Gln Leu Ser Arg Leu Asn Lys Thr Leu        46


FIG. 2
```

EP 0 412 912 B1

```
GCA GCT GGT CGT CCA ACT ATT AAC CAC CCA ACC TTT GTA      251
Ala Ala Gly Arg Pro Thr Ile Asn His Pro Thr Phe Val       59


GGG AGT GAA CGC TGT AGA CCT GGG TAC ACG TTC ACA TCT      290
Gly Ser Glu Arg Cys Arg Pro Gly Tyr Thr Phe Thr Ser       72


ATT ACC CTA AAG CCA CCA AAA ATA GAC CGT GGG TCT TAT      329
Ile Thr Leu Lys Pro Pro Lys Ile Asp Arg Gly Ser Tyr       85


TAC GGT AAA AGG TTG TTA CTA CCT GAT TCA GTC ACG GAA      368
Tyr Gly Lys Arg Leu Leu Leu Pro Asp Ser Val Thr Glu       98
```

FIG. 2 - Suite 1

EP 0 412 912 B1

```
TAT GAT AAG AAG CTT GTT TCG CGC ATT CAA ATT CGA GTT    407
Tyr Asp Lys Lys Leu Val Ser Arg Ile Gln Ile Arg Val    111


AAT CCT TTG CCG AAA TTT GAT TCT ACC GTG TGG GTG ACA    446
Asn Pro Leu Pro Lys Phe Asp Ser Thr Val Trp Val Thr    124


GTC CGT AAA GTT CCT GCC TCC TCG GAC TTA TCC GTT GCC    485
Val Arg Lys Val Pro Ala Ser Ser Asp Leu Ser Val Ala    137


GCC ATC TCT GCT ATG TTC GCG GAC GGA GCC TCA CCG GTA    524
Ala Ile Ser Ala Met Phe Ala Asp Gly Ala Ser Pro Val    150
```

FIG. 2 - Suite 2

EP 0 412 912 B1

```
CTG GTT TAT CAG TAT GCC GCA TCT GGA GTC CAA GCC AAC    563
Leu Val Tyr Gln Tyr Ala Ala Ser Gly Val Gln Ala Asn    163


AAC AAA CTG TTG TAT GAT CTT TCG GCG ATG CGC GCT GAT    602
Asn Lys Leu Leu Tyr Asp Leu Ser Ala Met Arg Ala Asp    176


ATA GGT GAC ATG AGA AAG TAC GCC GTC CTC GTG TAT TCA    641
Ile Gly Asp Met Arg Lys Tyr Ala Val Leu Val Tyr Ser    189


AAA GAC GAT GCG CTC GAG ACG GAC GAG CTA GTA CTT CAT    680
Lys Asp Asp Ala Leu Glu Thr Asp Glu Leu Val leu His    202
```

FIG. 2 - Suite 3

EP 0 412 912 B1

```
GTT GAC ATC GAG CAC CAA CGC ATT CCC ACA TCT GGA GTG       719
Val Asp Ile Glu His Gln Arg Ile Pro Thr Ser Gly Val       215


CTC CCA GTC TGATTCCGTGTTCCCAGAATCCTCCCTCCGATCTCTGTGG       768
Leu Pro Val                                               218


CGGGAGCTGAGTTGGCAGTTCTGCTATAAACTGTCTGAAGTCACTAAACGTT      820


TTTTACGGTGAACGGGTTGTCCATCCAGCTTACGGCTAAAATGGTCAGTCGT      872


GGAGAAATCCACGCCAGCAGATTTACAAATCTCTGAGGCGCCTTTGAAACCA      924


TCTCCTAGGTTTCTTCGGAAGGACTTCGGTCCGTGTACCTCTAGCACAACGT      976
```

FIG. 2 - Suite 4

EP 0 412 912 B1

```
AGAGAGTGTGTGTGCTGTGTTTTCTCTTTTGTGTCGTAGAATTGAGTCGAG        51

TC ATG GAC AAA TCT GAA TCA ACC AGT GCT GGT CGT AAC          89
   Met Asp Lys Ser Glu Ser Thr Ser Ala Gly Arg Asn         12

CGT CGA CGT CGT CCG CGT CGT GGT TCC CGC TCC GCC CCC        128
Arg Arg Arg Arg Pro Arg Arg Gly Ser Arg Ser Ala Pro        25

TCC TCC GCG GAT GCT AAC TTT AGA GTC TTG TCG CAG CAG        167
Ser Ser Ala Asp Ala Asn Phe Arg Val Leu Ser Gln Gln        38

CTT TCG CGA CTT AAT AAG ACG TTA GCA GCT GGT CGT CCA        206
Leu Ser Arg Leu Asn Lys Thr Leu Ala Ala Gly Arg Pro        51
```

FIG. 3

EP 0 412 912 B1

```
ACT ATT AAC CAC CCA ACC TTT GTA GGG AGT GAA CGC TGT    245
Thr Ile Asn His Pro Thr Phe Val Gly Ser Glu Arg Cys     64

AGA CCT GGG TAC ACG TTC ACA TCT ATT ACC CTA AAG CCA    284
Arg Pro Gly Tyr Thr Phe Thr Ser Ile Thr Leu Lys Pro     77

CCA AAA ATA GAC CGT GGG TCT TAT TAC GGT AAA AGG TTG    323
Pro Lys Ile Asp Arg Gly Ser Tyr Tyr Gly Lys Arg Leu     90

TTA CTA CCT GAT TCA GTC ACG GAA TAT GAT AAG AAG CTT    362
Leu Leu Pro Asp Ser Val Thr Glu Tyr Asp Lys Lys Leu    103

GTT TCG CGC ATT CAA ATT CGA GTT AAT CCT TTG CCG AAA    401
Val Ser Arg Ile Gln Ile Arg Val Asn Pro Leu Pro Lys    116
```

FIG. 3 SUITE 1

EP 0 412 912 B1

```
TTT GAT TCT ACC GTG TGG GTG ACA GTC CGT AAA GTT CCT     440
Phe Asp Ser Thr Val Trp Val Thr Val Arg Lys Val Pro     129

GCC TCC TCG GAC TTA TCC GTT GCC GCC ATC TCT GCT ATG     479
Ala Ser Ser Asp Leu Ser Val Ala Ala Ile Ser Ala Met    142

TTC GCG GAC GGA GCC TCA CCG GTA CTG GTT TAT CAG TAT     518
Phe Ala Asp Gly Ala Ser Pro Val Leu Val Tyr Gln Tyr    155

GCC GCA TCT GGA GTC CAA GCC AAC AAC AAA CTG TTG TAT     557
Ala Ala Ser Gly Val Gln Ala Asn Asn Lys Leu Leu Tyr    168


GAT CTT TCG GCG ATG CGC GCT GAT ATA GGT GAC ATG AGA     596
Asp Leu Ser Ala Met Arg Ala Asp Ile Gly Asp Met Arg    181
```

FIG. 3 SUITE 2

EP 0 412 912 B1

```
AAG TAC GCC GTC CTC GTG TAT TCA AAA GAC GAT GCG CTA       635
Lys Tyr Ala Val Leu Val Tyr Ser Lys Asp Asp Ala Leu       194

GAG ACG GAC GAG CTA GTA CTT CAT GTT GAC ATC GAG CAC       674
Glu Thr Asp Glu Leu Val Leu His Val Asp Ile Glu His       207

CAA CGC ATT CCC ACG TCT GGA GTG CTC CCA GTC TGATTCGT      715
Gln Arg Ile Pro Thr Ser Gly Val Leu Pro Val               218

GTTCCCAGAATCCTCCCTCCGATCTCTGTGGCGGGAGCTGAGTTGGCAGTTC      767

TGCTATAAACTGTCTGAAGTCACTAAACGTTTTTACGGTGAACGGGTTGTCC      819
```

FIG. 3 SUITE 3

EP 0 412 912 B1

ATCCAGCTTACGGCTAAAATGGTCAGTCGTGGAGAAATCCACGCCAGTAGAT 871

TTACAAATCTCTGAGGCGCCTTTGAAACCATCTCCTAGGTTTCTTCGGAAGG 923

ACTTCGGTCCGTGTACCTCTAGCACAACGTGCTAGTTTCAGGGTACGGGTGC 975

CCCCCCACTTTCGTGGGGGCCTCCAAAAGGAG 1007

FIG. 3 SUITE 4

EP 0 412 912 B1

FIG. 4 : Influence de la concentration de CaCl$_2$
sur l'induction de bourgeons chez des
melons de génotypes différents.

FIG. 5

photo 1.

MILIEU B6

| | | |
|---|---|---|
| $KH_2PO_4$ | 85 | mg/l |
| $MgSO_4. 7H_2O$ | 185 | |
| $CaCl_2.2H_220$ | 1720 | |
| $KNO_3$ | 950 | |
| $NH_4NO_3$ | 165 | |
| $MnSO_4.H_2O$ | 16,9 | |
| KI | 0,83 | |
| $H_3BO_3$ | 6,2 | |
| $ZnSO_4.7H_2O$ | 8,6 | |
| $CuSO_4.5H_2O$ | 0,025 | |
| $Na_2MoO_4.2 H_2O$ | 0,25 | |
| $CoCl_2.6H_2O$ | 0,025 | |
| $FeSO_4.7H_2O$ | 27,85 | |
| $Na_2EDTA$ | 37,25 | |
| acide nicotinique | 0,50 | |
| Thiamine-HCl | 0,10 | |
| pyridoxine-HCl | 0,50 | |
| Glycine | 2,0 | |
| myo-inositol | 100 | |
| saccharose | 10 000 | |
| gelrite | 4 000 | |
| pH 5,6 | | |

TABLEAU I